# EUROPÄISCHE PATENTSCHRIFT

(11) **EP 4 376 732 B1**
(45) Veröffentlichungstag und Bekanntmachung des Hinweises auf die Patenterteilung: **05.03.2025**
(21) Anmeldenummer: 22757867.1
(22) Anmeldetag: 25.07.2022
(51) Int. Cl.: A61B 17/29, A61B 34/00, A61B 17/00

(54) **CHIRURGISCHES INSTRUMENT UND LENKGETRIEBE DAFÜR**
SURGICAL INSTRUMENT AND STEERING MECHANISM FOR SAME
INSTRUMENT CHIRURGICAL ET SON MÉCANISME DE DIRECTION

(30) Priorität: 28.07.2021 DE 102021119536
(43) Veröffentlichungstag der Anmeldung: 05.06.2024
(73) Patentinhaber: Karl Storz SE & Co. KG, 78532 Tuttlingen (DE)
(72) Erfinder: GRÜNER, Sven Axel, 78532 Tuttlingen (DE)
(86) Internationale Anmeldenummer: PCT/EP2022/070834
(87) Internationale Veröffentlichungsnummer: WO 2023/006684

(56) Entgegenhaltungen:
- DE-A1- 102019 121 092
- US-A- 5 454 827
- US-A1- 2010 228 284

## Beschreibung

Die Erfindung betrifft ein Lenkgetriebe eines chirurgischen Instruments zur Abwinkelung einer Werkzeugspitze mittels einer räumlich ausrichtbaren Taumelscheibe, sowie ein chirurgisches Instrument, das ein solches Lenkgetriebe aufweist.

Aus dem Stand der Technik sind chirurgische Instrumente bekannt, die manuell oder von einem Roboter geführt werden können und die Werkzeuge aufweisen, deren Werkzeugspitze mittels mehrerer ineinandergreifender Schwenkglieder verschwenkt werden kann. Diese Schwenkglieder sind mit einer Vielzahl Lenkdrähte oder -seile verbunden, um eine feinfühlige Steuerung der Werkzeugspitze zu erreichen. Mit vielen dünnen Lenkdrähten gegenüber wenigen dickeren Lenkdrähten kann eine gleichmäßigere Kraftverteilung in alle Abwinklungsrichtungen erzielt werden.

Aus US 5,454,827 B2 ist bekannt, solche Lenkdrähte mit einer proximalseitig in einer Betätigungseinheit angeordneten räumlich verstellbaren Scheibe zu koppeln, die über eine Stange mit einem manuell betätigbaren Steuerhebel verbunden ist, sodass eine Bewegung der räumlich verstellbaren Taumelscheibe eine entsprechende relative Bewegung der distalseitigen Schwenkglieder und somit ein Verschwenken der Werkzeugspitze verursacht.

Die Ausbildung des Antriebs für die Lenkdrähte mit der räumlich verstellbaren Taumelscheibe, an der alle vier Lenkdrähte gelagert sind, hat den Vorteil, dass dies eine räumlich kompakte Bauweise ermöglicht und nur ein Bauteil bewegt werden muss, um alle Lenkdrähte ansprechen zu können. Diese Konstruktion bringt mit sich, dass die Verwendung einer nur geringen Anzahl von Lenkdrähten möglich ist, und dass die als Antrieb für die Lenkdrähte dienende räumlich verstellbare Scheibe manuell zu betätigen ist, was beides die Feinfühligkeit und Reproduzierbarkeit der Verstellung der distalseitigen Schwenkglieder beeinflusst.

In der US 7,699,855 B2 ist ein chirurgisches Instrument offenbart, das eine Schnittstelle aufweist, um das Instrument mit einem Roboter-Arm verbinden zu können. Dabei sind alle Antriebe, die das Instrument steuern, in dem Roboter-Arm angeordnet. Die Übertragung der Drehwinkel von Antrieben zum Instrument erfolgt über Kupplungsscheiben in einer gemeinsamen Trenn-Ebene.

Ferner ist bekannt, in einem chirurgischen Instrument mit einem kompakten Lenkgetriebe die Stellwinkel von zwei Antrieben direkt auf die Taumelscheibe zu übertragen, um diese zur Steuerung der Werkzeugspitze auszurichten. Dazu werden an der Taumelscheibe Lenkdrähte befestigt, sodass sich die Werkzeugspitze durch Ausrichtung der Taumelscheibe stufenlos und flüssig steuern lässt. Dazu weist das bekannte Lenkgetriebe zwei um 180° zueinander versetzte Antriebskegelräder auf, die auf einer gemeinsamen Drehachse angeordnet sind, die senkrecht zu einer Instrumentenlängsachse verläuft, mit jeweils einem zugeordneten Motor. Die Taumelscheibe ist zwischen den Antriebskegelrädern angeordnet und in einem Lenkring gelagert, der drehfest mit einem dritten Kegelrad verbunden ist, das mit den beiden Antriebskegelrädern in Eingriff steht und um eine Drehachse drehbar ist, die senkrecht zu der Instrumentenlängsachse und senkrecht zu der gemeinsamen Drehachse der Antriebskegelräder verläuft. Die Verzahnungskette wird durch ein viertes Kegelrad ergänzt, das auf der Drehachse des dritten Kegelrads um 180° versetzt zum dritten Kegelrad angeordnet ist und mit den beiden Antriebskegelrädern in Eingriff steht, wobei der Lenkring frei drehbar im vierten Kegelrad gelagert ist. Die auf diese Weise geschlossene Verzahnungskette sichert den Eingriff aller Kegelräder miteinander und ermöglicht eine gleichmäßig umlaufende Kraftverteilung.

Die Ausbildung des Antriebs für die Lenkdrähte mit der räumlich verstellbaren Taumelscheibe, an der alle Lenkdrähte gelagert sind, hat den Vorteil, dass dies eine räumlich kompakte Bauweise ermöglicht und nur ein Bauteil bewegt werden muss, um alle Lenkdrähte ansprechen zu können.

Die WO 2014/004 242 beschreibt ebenfalls eine solche Schnittstelle, wobei die Antriebe in dem Roboter-Arm verbaut sind. Die vorstehende Konstruktion ist verbunden mit einem komplexen Aufbau und einer indirekten Ansteuerung. Die Antriebe sind nicht in dem chirurgischen Instrument direkt angeordnet, womit die Ansteuerung der Taumelscheibe nicht linear erfolgt.

Auch US 10,105,128 B2 offenbart eine Ansteuerung einer solchen Werkzeugspitze; dort erfolgt dies über eine Mechanik, die Zahnscheiben-Segmente und Gelenkstangen umfasst, um die Bewegung der Antriebe auf die Taumelscheibe zu übertragen.

Die DE 10 2019 121 092 A1 beschreibt ein Lenkgetriebe mit Taumelscheibe und zwei Motoren, die die Taumelscheibe bewegen. Die US 2010/228284 A1 beschreibt Mechanismen zum Steuern eines Gelenks in der chirurgischen Robotik. Insbesondere offenbaren die DE 102019 121 092 A1 und die US 2010/228284 A1 den Oberbegriff des Anspruchs 1. Ausgehend von diesem Stand der Technik ist es Aufgabe der vorliegenden Erfindung, eine alternative Ansteuerung und Führung des Lenkrings bereitzustellen.

Diese Aufgabe wird durch ein Lenkgetriebe mit den Merkmalen des Anspruchs 1 gelöst. Die weitere Aufgabe der Bereitstellung eines chirurgischen Instruments mit einer alternativen Führung des Lenkrings wird durch das chirurgische Instrument mit den Merkmalen des abhängigen Anspruchs 9 gelöst.

Weiterbildungen des Lenkgetriebes und des chirurgischen Instruments sind in den jeweiligen Unteransprüchen ausgeführt.

Eine erste Ausführungsform des Lenkgetriebes ist für ein chirurgisches Instrument ausgebildet, das am proximalen Ende eines Schafts angeordnet werden kann, der eine Längsachse B definiert und am distalen Ende eine Abwinkelungsmechanik aufweist. Dabei hat das Lenkgetriebe zwei motorisierte Antriebe und ist dazu ausgebildet, über die Stellwinkel der zwei Antriebe eine Taumelscheibe räumlich auszurichten. Die Taumelscheibe ist ferner dazu ausgebildet, die distale Abwinkelungsmechanik des chirurgischen Instruments zu steuern.

Erfindungsgemäß ist die Taumelscheibe in einem Lenkring angeordnet. Der erste der zwei motorisierten Antriebe weist eine von einem ersten Motor angetriebene erste Antriebswelle auf, die mit dem Lenkring über einen ersten Kraftübertrager direkt in Wirkverbindung steht, indem der erste Kraftübertrager unmittelbar den Lenkring an einem ersten Wirkabschnitt kontaktiert. Hierzu ist der erste Kraftübertrager auf der ersten Antriebswelle angeordnet, die eine erste Antriebssachse C definiert. Ferner hat der zweite der zwei motorisierten Antriebe eine von einem zweiten Motor angetriebene zweite Antriebswelle, die mit dem Lenkring über einen zweiten Kraftübertrager direkt in Wirkverbindung steht, indem der zweite Kraftübertrager unmittelbar den Lenkring an einem zweiten Wirkabschnitt kontaktiert. Dabei ist der zweite Kraftübertrager auf der zweiten Antriebswelle angeordnet, die eine zweite Antriebesachse C' definiert. Der Lenkring ist dabei um ein kardanisches Zentrum kardanisch gelagert, sodass eine Bewegungsführung in zwei Raumachsen ermöglicht wird, wodurch die Werkzeugspitze gezielt gesteuert werden kann. Damit der Eingriff mit den Kraftübertragern auch beim Verkippen des Lenkrings bestehen bleibt, ist der Lenkring zumindest an dem ersten und zweiten Wirkabschnitt um das kardanische Zentrum sphärisch bzw. kugelsegmentförmig ausgebildet. Die kugelsegmentförmigen Wirkabschnitte weisen einen gemeinsamen Kugelmittelpunkt auf, der dem kardanischen Zentrum entspricht. Auf diese Weise schneiden sich eine erste Kippachse E, die durch einen Kontaktpunkt des ersten Kraftübertragers mit dem Lenkring und das kardanische Zentrum definiert wird, und eine zweite Kippachse E', die durch einen Kontaktpunkt des zweiten Kraftübertragers mit dem Lenkring und das kardanische Zentrum definiert wird, im rechten Winkel in dem kardanischen Zentrum. Das heißt, dass die Antriebe den Lenkring mit den Wirkabschnitten so berühren, dass sich die als Kippachsen E, E' definierten Normalen der sich in den jeweiligen Kontaktpunkten berührenden Oberflächen im kardanischen Zentrum senkrecht schneiden.

Als "kugelsegmentförmig" werden Formen verstanden, die als Teil eines Kugelkörpers definiert werden können, der durch den Schnitt mit einer Ebene abgetrennt wird und dessen gekrümmte Oberfläche als Kugelkalotte bezeichnet wird. Vorliegend sollen unter "kugelsegmentförmig" auch Kugelscheibenformen umfasst sein, die als Teil eines Kugelkörpers entstehen, bei dem zwei Kugelsegmente ("Polkappen") durch parallelen Schnittebenen entfernt wurden, wobei die gekrümmte Oberfläche der Kugelscheibe als Kugelzone bezeichnet wird. Beispielsweise kann der Lenkring eine Kugelscheibenform aufweisen, bei der die beiden parallelen Schnittebenen äquidistant bzw. symmetrisch zur Durchmesserebene verlaufen, sodass die Lenkringoberfläche als eine zur Durchmesserebene symmetrische Kugelzone ausgebildet ist.

Der Lenkring hat damit zumindest im Bereich der Wirkabschnitte sphärischen bzw. teilsphärischen Charakter, wobei ein Kreismittelpunkt einer Kugel, die durch die sphärischen bzw. teilsphärischen Wirkabschnitte definiert wird, das kardanische Zentrum ist. Grundsätzlich ist es dazu ausreichend, wenn die Oberfläche des Lenkrings nur an den zwei Wirkabschnitten sphärisch ausgebildet ist, die mit den Kraftüberträgern in Eingriff kommen.

Allen nachfolgend beschriebenen Varianten des Kraftübertragers ist dieses Grundprinzip gemeinsam: In den Kontaktpunkten zwischen Kraftübertrager und Lenkring ist die Bewegung des Kraftübertragers bei infinitesimaler Betrachtung in diesem Punkt immer parallel zur Hauptachse B des chirurgischen Instruments und in Bezug auf die Hauptachse B nach vorne oder hinten gerichtet, je nach Bewegungsrichtung des Kraftübertragers. Diese Bewegung wird auf den Lenkring übertragen. Durch dessen kardanische Aufhängung ergibt sich so eine Verkippung des Lenkrings um eine oder beide Kippachsen E, E'.

"Kraftübertrager" meint hierbei jedes Bauteil, das die von Motoren initiierte Bewegung, egal ob rotatorisch oder linear, direkt aufnimmt und auf den Wirkabschnitt des Lenkrings weitergeben kann.

"Wirkabschnitt" ist hierbei der Bereich des Lenkrings, der mit dem Kraftübertrager eine kraftübertragende Wirkverbindung eingehen kann, also direkt in Kontakt mit dem Kraftübertrager ist, bspw. durch eine Reibschluss-Wirkverbindung mittels Reibelemente, oder in Eingriff steht, bspw. durch eine Verzahnung oder andere geeignete kraftübertragende Wirkverbindungen.

Vorteilhaft kann der Lenkring, der die Taumelscheibe umfasst, mittels der Kraftübertrager von den Antriebsmotoren direkt angesteuert werden. Es sind keine weiteren Umlenkmechanismen oder Getriebeübersetzungen notwendig, so dass die kürzest mögliche Übertragungskette ermöglicht wird. Eine solche direkte kurze Kraftübertragung ist vorteilhaft mit geringem Spiel und einer Reduktion von Übertragungs- und Reibungsverlusten verbunden, und ermöglicht zudem aufgrund des lineares Übertragungsverhaltens eine einfache softwaretechnische Steuerung, um das zu steuernde Bauteil präzise zu steuern.

Eine weitere Ausführungsform des erfindungsgemäßen Lenkgetriebes sieht vor, dass die kardanische Lagerung des Lenkrings bereitgestellt wird, indem
a) der Lenkring an einer ersten Befestigungsvorrichtung kardanisch aufgehängt ist. Die Befestigungsvorrichtung ist ein Bügel, der an einem Abschnitt des Lenkrings angeordnet ist, der von den Wirkabschnitten abgewandt ist. Ferner ist der Bügel beidenends an dem Gehäuse schwenkbar um eine Schwenkachse A gelagert, die senkrecht zu der Längsachse B und senkrecht zu den Antriebsachsen C, C' verläuft. Der Bügel weist mittig eine Aufnahmeöffnung auf, wobei der Lenkring in der Aufnahmeöffnung drehbar um eine Drehachse D gelagert ist, die senkrecht zu der Schwenkachse A verläuft. Die Taumelscheibe kann dabei vorzugsweise drehbar in dem Lenkring gelagert sein, um auch eine Rotationsbewegung der Taumelscheibe in dem Lenkring zuzulassen, gegebenenfalls aber auch fest mit dem Lenkring verbunden oder auch einteilig mit dem Lenkring sein, so dass ein kombiniertes Bauteil aus Lenkring und Taumelscheibe entsteht.

Die drehbare Lagerung des Bügels an dem Gehäuse umfasst Lagerstifte, mittels derer der Bügel beidseitig um die Schwenkachse A drehbar in dem Gehäuse gelagert ist, so dass der Lenkring, der sich ausschließlich um seine Quer- und Hochachse, umfassend entsprechend auch Überlagerungen, drehen kann, in seiner räumlichen Position definiert ist und eine Drehung um die Hauptachse B unterbunden wird. Die genannte kardanische Aufhängung des Lenkrings mittels Bügel ist konstruktiv einfach und kann präzise gesteuert werden. Zudem ist der Lenkring, indem der Bügel diesen überspannt, zusätzlich gehaust und geschützt.

Alternativ zu der kardanischen Aufhängung mittels Bügel kann die kardanische Lagerung des Lenkrings bereitgestellt werden, indem
b) der Lenkring über die Taumelscheibe, die um die Längsachse B rotierbar in dem Lenkring gelagert oder fest mit dem Lenkring verbunden ist, kardanisch auf einer koaxial zu der Längsachse B des Schaftes verlaufenden Hauptwelle gelagert ist. Dazu ist die Taumelscheibe über zwei um 180° versetzt zueinander, d. h. diametral und koaxial, angeordnete Lagerstifte verschwenkbar auf einer Kreuzgelenkscheibe gelagert, welche wiederum über zwei um 180° versetzt zueinander, d. h. diametral und koaxial, angeordnete Lagerstifte verschwenkbar auf der Hauptwelle gelagert ist, wobei die Lagerstift-Paare der Taumelscheibe und der Kreuzgelenkscheibe um 90° versetzt zueinander angeordnet sind.

Damit der Lenkring sich auch hier ausschließlich um die Quer- und Hochachse inklusive Überlagerungen drehen kann und seine Position im Raum fixiert und eine Drehung um die Hauptachse B unterbunden wird, kann der Lenkring optional in Bezug auf die Längsachse B drehfest mit einem Gehäuse gekoppelt sein, etwa indem ein sich von dem Lenkring radial nach außen erstreckender Stift, beispielsweise an der Unterseite des Lenkrings zwischen den Wirkabschnitten, in einer Nut des Gehäuses geführt wird, die sich parallel zur Längsachse B entlang dem Bewegungsradius des Stifts erstreckt.

Wird durch den ersten Motor die erste Antriebswelle bewegt und mit ihr der erste Kraftübertrager um die Antriebsachse C gedreht, so wird auf Grund der Wirkverbindung zwischen Lenkring und dem ersten Kraftübertrager diese Bewegung an dem ersten Wirkabschnitt auf den Lenkring übertragen. Bleibt der zweite Kraftübertrager währenddessen stehen, so wird der Lenkring in seinem Kontaktpunkt mit dem ersten Kraftübertrager bewegt und bleibt im Kontaktpunkt des zweiten Wirkabschnitts mit dem zweiten Kraftübertrager stehen, wodurch der Lenkring verkippt wird und zwar um die zweite Kippachse E', die durch den fixen Kontaktpunkt des zweiten Wirkabschnitts mit dem zweiten Kraftübertrager führt und in einem Winkel von 45° zu sowohl der Drehachse D als auch der Drehachse A liegt. Umgekehrt bewirkt eine Drehung des zweiten Kraftübertragers um die Antriebsachse C' eine Drehung des Lenkrings um die erste Kippachse E, die analog zu der zweiten Kippachse E' in einem Winkel von 45° zu sowohl der Drehachse D als auch der Drehachse A liegt und diesmal durch den fixen Kontaktpunkt von dem erstem Kraftübertrager und dem ersten Wirkabschnitt des Lenkrings verläuft. Drehen beide Kraftübertrager, überlagern sich die Verkippungen und der Lenkring, und damit die Taumelscheibe, kann im Raum um beide Kippachsen E, E', also zweidimensional verkippt werden.

Eine weitere Ausführungsform des erfindungsgemäßen Lenkgetriebes sieht vor, dass die Wirkverbindung einen Eingriff bildet, indem der erste und der zweite Kraftübertrager rotatorisch gelagerte Antriebskegel mit einem Kegelkopf sind, der umfänglich eine Vielzahl von Antriebsnoppen einen Noppenkranz bildend aufweist. Hierbei hat der Lenkring an den Wirkabschnitten Ausnehmungen, die mit den Antriebsnoppen der beiden Noppenkränze kämmen. Der Kegelkopf hat bevorzugt eine teil- oder volltorodiale Form, um einen guten Kontaktpunkt zu dem Lenkring zu erreichen.

Bevorzugt kann vorgesehen sein, dass die Noppen in einer weiteren Ausführungsform des erfindungsgemäßen Lenkgetriebes eine halbkugelige oder eine halbellipsoide Form haben.

Diese Formen ermöglichen ein sicheres Kämmen mit dem Wirkabschnitt des Lenkringes sowie eine gute Kraftübertragung von dem jeweiligen Kraftübertrager auf den Lenkring. Die Ausnehmungen sind korrespondierend zu der Form der Antriebsnoppen entweder konzentrisch angeordnete Ringnuten oder bogenförmige Nuten, die quasi eine Hypoidähnliche Zahnung bilden. Die Ringe werden am besten von halbkugeligen Noppen und die bogenförmigen Nuten am besten von halbellipsoiden Noppen gekämmt. Die Kraftübertragung erfolgt in beiden Fällen ohne Ruckeln und reibungsarm. Durch den Eingriff der Noppen in die Ausnehmungen können große Antriebskräfte exakt übertragen werden, ohne dass ein Durchschlupf auftritt.

In einer alternativen Ausführungsform des erfindungsgemäßen Lenkgetriebes können der erste und der zweite Kraftübertrager Schneckenwellen sein, die parallel zu der Längsachse B verlaufen. Dazu weist der Lenkring an seinen Wirkabschnitten konzentrisch angeordnete Ausnehmungen/Ringnuten auf, die mit der jeweiligen Schneckenwelle kämmen.

Die beiden vorgenannten Ausführungsformen bieten formschlüssige Wirkverbindungen, bei denen die beiden Bauteile Lenkring und Kraftübertrager in einer Art Verzahnung in Eingriff miteinander stehen. Hiermit ist eine gute und präzise Kraftübertragung möglich, die dann eine präzise Ansteuerung der Taumelscheibe ermöglicht.

Eine noch einer weitere, alternativen Ausführungsform des erfindungsgemäßen Lenkgetriebes sieht vor, dass die Wirkverbindung durch Reibung bereitgestellt wird. Diese erfolgt, indem der erste und der zweite Kraftübertrager rotatorisch gelagerte Antriebskegel mit einem Kegelkopf sind, der umfänglich ein Reibelement aufweist. Das Reibelement ist bevorzugt ein um den Kegelkopf aufgebrachtes Material, das mit dem Wirkabschnitt des Lenkrings eine höhere Reibung bereitstellt als das Material, aus dem der Kegelkopf besteht. Ein solches reibungserhöhendes Material kann zum verbesserten Halt am Kegelkopf auch in einer um den Kegelkopf umlaufenden Nut eingebracht sein. Alternativ kann das Reibelement ein um den Kegelkopf umfänglich angelegtes Reibband sein, das ein Gummiband oder ein Band mit aufgerauter Oberfläche oder ein Band mit einer Zahnung, einer Mikro-Verzahnung oder Noppen ist.

"Reibung" meint hiermit eine Wirkverbindung, die sich reibschlüssig bildet, wobei die Reibungskraft zwischen zwei Bauteilen, die sich berühren (hier Lenkring und Antriebskegel), so groß ist, dass wenn der Antriebskegel in Rotation versetzt wird, dieser auf dem Lenkring quasi abrollt und ihn dadurch in Bewegung versetzt. "Reibelement" meint in diesem Zusammenhang jedes Bauteil, das eine geforderte Materialeigenschaft mitbringt, so p einen erhöhten Reibungswiderstand (bspw. Gleitreibungskraft). Dies kann durch eine bestimmte Materialwahl oder Oberflächenbeschaffenheit, wie die oben genannte Aufrauhung oder Zahnung, erzielt werden. Die bei einer reibschlüssigen Verbindung zwangsläufig nicht vollkommen ausschließbare Schlupfgefahr kann durch einen erhöhten Anpressdruck reduziert werden, der über die Positionierung der Kraftübertrager in Bezug auf den Lenkring bestimmt wird.

Der Vorteil dieser Variante ist, dass durch die reibschlüssige Wirkverbindung eine nahezu stetige Verkippung des Lenkrings, also stufenlos, erzielt werden kann. Damit kann ebenfalls eine präzise Steuerung der Taumelscheibe erreicht werden. Weitere Vorteile dieser Variante sind eine absolute Spielfreiheit und günstige Bauteile bzw. Herstellung.

Um die jeweiligen Vorteile der formschlüssigen und reibschlüssigen Wirkverbindung zu kombinieren, bzw. die jeweiligen Nachteile zu minimieren, sind auch Mischformen denkbar: In einer weiteren Ausführungsform ist daher vorgesehen, dass die Wirkverbindung des ersten Kraftübertragers einen formschlüssigen Eingriff bereitstellt und die Wirkverbindung des zweiten Kraftübertragers durch Reibung bereitgestellt wird. Dabei kann der erste Kraftübertrager ein rotatorisch gelagerter Antriebskegel mit einem Kegelkopf sein, der umfänglich eine Vielzahl von Antriebsnoppen einen Noppenkranz bildend aufweist, oder eine Schneckenwelle, die parallel zu der Längsachse B verläuft. Der Lenkring weist entsprechend an dem ersten Wirkabschnitt Ausnehmungen auf, die mit den Antriebsnoppen der beiden Noppenkränze oder der Schneckenwelle kämmen. Der zweite Kraftübertrager, der ebenfalls ein rotatorisch gelagerter Antriebskegel mit einem Kegelkopf ist, weist am Kegelkopf umfänglich ein Reibelement auf, bevorzugt ein um den Kegelkopf aufgebrachtes Material, das mit dem zweiten Wirkabschnitt Lenkring eine höhere Reibung bereitstellt als das Material, aus dem der Kegelkopf besteht. Alternativ kann das Reibelement ein um den Kegelkopf umfänglich angelegtes Reibband sein, das ein Gummiband oder ein Band mit aufgerauter Oberfläche oder ein Band mit einer Zahnung oder Noppen ist. Indem ein Antriebskegel mit formschlüssiger Verzahnung und der anderen reibschlüssig ausgebildet wird, werden folglich Schlupf und Spiel minimiert. Als eine Variante dieser Ausführungsform können eine kombinierte formschlüssige und reibschlüssige Wirkverbindung auch gemeinsam auf einem Antriebskegel realisiert sein, indem z. B. dieser einen Noppenkranz aufweist, der zu dem Formschluss durch die Verzahnung mit den Ausnehmungen an dem Wirkabschnitt auch einen Reibschluss aufweist, indem die Oberfläche des Antriebskegels mit dem Noppenkranz oder die Oberfläche des Wirkabschnitts mit den Ausnehmungen zusätzlich mit einer Gummierung versehen ist und so das Spiel verhindert wird. Die zugrunde liegende Verzahnungskontur stellt aber trotzdem über die gesamte Lebensdauer noch den Formschluss sicher und verhindert Schlupf bei gleichzeitig hoher Kraftübertragung.

Ferner haben in einer weiteren Ausführungsform des erfindungsgemäßen Lenkgetriebes die rotatorisch gelagerten Antriebskegel jeweils einen Kegelschaft, um den benachbart zu dem Kegelkopf ein Lagerring angeordnet ist, der beispielsweise ein Rollen- oder ein Kugellager haust. Das Gehäuse weist dazu eine Basis mit jeweils einer Durchtrittsöffnung mit zwei Öffnungsabschnitten für jede Antriebsachse auf. Die beiden Öffnungsabschnitte der Durchtrittsöffnungen sind axial benachbart zueinander, so dass sie ineinander übergehen und dabei eine Schulter bilden, wobei sie sich oberhalb der Schulter zu dem Lenkring weisend auf einen dem Durchmesser der Lagerringe entsprechenden Durchmesser weiten. D. h., dass jede Durchtrittsöffnung einen ersten Öffnungsabschnitt mit einem Durchmesser hat, der einem Durchmesser des Lagerrings entspricht, und einen zum ersten Öffnungsabschnitt koaxialen zweiten Öffnungsabschnitt mit einem Durchmesser aufweist, der kleiner als der Durchmesser des ersten Öffnungsabschnitts ist, sodass das Gehäuse in jeder Durchtrittsöffnung eine Schulter bildet. Dann liegt jeder der Lagerringe auf der jeweiligen Schulter auf und wird sicher gehalten. Die Antriebswelle, die durch den Lagerring und damit auch beide Durchtrittsöffnungsabschnitte durchstößt, wird in ihrer Rotation seitlich geführt und gehalten. Dadurch kann die von den Motoren aufgebrachte Kraft über die Antriebswellen direkt auf die Antriebskegelräder übertragen werden.

Eine alternative Ausführungsform des erfindungsgemäßen Lenkgetriebes sieht vor, dass die Wirkverbindung einen Linearantrieb bildet, indem der erste und der zweite Kraftübertrager Zahnstangen mit einem Zahnungsabschnitt sind. Dabei weist der Lenkring an seinem Wirkabschnitt konzentrisch angeordnete Ausnehmungen auf, die mit den Zahnungsabschnitten der beiden Zahnstangen kämmen. In noch einer alternativen Ausführungsform des erfindungsgemäßen Lenkgetriebes könnte auch eine stoffschlüssige Wirkverbindung erreicht werden. Hierzu könnten Zugmittel wie Seile oder Bänder mit beiden Enden an den jeweiligen Wirkabschnitten des Lenkrings befestigt sein und einmal um den jeweiligen Kraftübertrager geschlungen werden. Diese Varianten bieten bei schmalen Gehäusen oder aus anderen konstruktiven Gründen erforderliche anders angeordnete Motoren eine direkte Ansteuerung der Taumelscheibe.

Eine erste Ausführungsform eines erfindungsgemäßen chirurgischen Instruments, das einen Schaft, eine am proximalen Ende des Schaftes angeordnete Betätigungseinheit und ein am distalen Ende des Schaftes angeordnetes Werkzeug mit einer mittels einer distalen Abwinkelungsmechanik abwinkelbaren Werkzeugspitze aufweist, die durch eine mittels zweier Antriebe räumlich ausrichtbare Taumelscheibe steuerbar ist, bezieht sich darauf, dass das chirurgische Instrument ein erfindungsgemäßes Lenkgetriebe zur räumlichen Ausrichtung der Taumelscheibe aufweist.

Durch das erfindungsgemäße Lenkgetriebe kann das chirurgische Instrument konstruktiv einfach und platzsparend aufgebaut werden, so dass eine einfache Verbindung zu einem Roboter-Arm ermöglicht werden kann, bei der die Bewegung der Antriebe direkt auf die Werkzeugspitze übertragen werden kann. Folge ist eine exakt steuerbare Verwendung des chirurgischen Instruments.

Um die räumlich verstellbare Taumelscheibe verstellen zu können, kann eine bevorzugte Ausführungsform des chirurgischen Instruments vorsehen, dass die Taumelscheibe kardanisch auf einer koaxial zu einer Längsachse B des Schaftes verlaufenden Hauptwelle gelagert ist. Der Lenkring ist dabei über die Taumelscheibe dann kardanisch gelagert, was auch als innere kardanische Lagerung bezeichnet werden kann. Dies ist eine alternative Ausführungsform im Vergleich zu einer äußeren kardanischen Lagerung des Lenkrings über den Bügel. Diese Lagerung kann je nach Bauweise und zur Verfügung stehendem Bauraum des chirurgischen Instruments vorteilhaft sein.

In einer weiteren Ausführungsform des erfindungsgemäßen chirurgischen Instruments ist das Betätigungselement axial verschiebbar in dem Schaft gelagert ist und steht proximalseitig mit der Betätigungseinheit in Wirkverbindung. Die distale Abwinkelungsmechanik der abwinkelbaren Werkzeugspitze besteht aus an dem distalen Ende des Schaftes angeordneten Schwenkgliedern, die über die in Längsrichtung des Schaftes verlaufenden Lenkdrähte mit dem Lenkgetriebe verbunden sind. Die Lenkdrähte sind an der Taumelscheibe befestigt, beispielsweise mittels einer Klemmverbindung, damit in einem Fall der Beschädigung die Lenkdrähte einfach ausgetauscht werden können. Diese Klemmverbindung kann bspw. durch Madenschrauben, die in radial eingebrachte Bohrungen in der Taumelscheibe bereitgestellt werden. Die Lenkdrähte können aber auch weniger aufwändig und daher kostengünstiger unlösbar an der Taumelscheibe befestigt sein, beispielsweise durch Schweißen oder Kleben, was bei einmalig zu benutzenden chirurgischen Instrumenten bevorzugt sein kann. Vorteilhaft an dieser Konstruktion gegenüber bekannten Konstruktionen ist, dass nicht nur ist die Verwendung einer geringen Anzahl von Lenkdrähten, nämlich von nur vier Lenkdrähten, und eine ausschließlich manuelle Betätigbarkeit der als Antrieb für die Lenkdrähte dienenden räumlich verstellbaren Scheibe möglich ist, sondern dass eine Vielzahl an Lenkdrähten frei gewählt werden und dadurch eine feinfühlige und reproduzierbare Verstellung der distalseitigen Schwenkglieder möglich ist.

Das erfindungsgemäße chirurgische Instrument hat den Vorteil, dass viele dünne Lenkdrähte zur Ansteuerung der verschwenkbaren Werkzeugspitze verwendet werden können und diese Ansteuerung aufgrund des motorisierten Antriebs für die räumlich verstellbare Scheibe, an der die Lenkdrähte proximalseitig gelagert sind, feinfühlig, exakt und reproduzierbar erfolgt.

Weitere Ausführungsformen des Lenkgetriebes und des chirurgischen Instruments sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung. Die Zeichnungen und die Beschreibung enthalten zahlreiche Merkmale in Kombination. Der Fachmann wird die Merkmale zweckmäßigerweise auch einzeln betrachten und zu sinnvollen weiteren Kombinationen unter Einhaltung des Geltungsbereichs der Ansprüche zusammenfassen.

Dabei zeigen:
- Fig. 1: eine perspektivische Ansicht des chirurgischen Instruments mit schematisch dargestellter Betätigungseinheit,
- Fig. 2: eine perspektivische Detailansicht einer ersten Ausführungsform des erfindungsgemäßen Lenkgetriebes ohne Kraftübertrager mit einem Teil eines Gehäuses,
- Fig. 3: eine perspektivische Detailansicht der ersten Ausführungsform des erfindungsgemäßen Lenkgetriebes mit Kraftübertrager ohne Gehäuse,
- Fig. 4: eine Schnittansicht des erfindungsgemäßen Lenkgetriebes gemäß der ersten Ausführungsform mit Kraftübertrager und Gehäuse,
- Fig. 5: eine perspektivische Detailansicht noch einer weiteren Ausführungsform des erfindungsgemäßen Lenkgetriebes mit Gummiring,
- Fig. 6: eine perspektivische Detailansicht noch einer weiteren Ausführungsform des erfindungsgemäßen Lenkgetriebes mit Antriebsköpfen mit Noppen,
- Fig. 7: eine perspektivische Detailansicht noch einer weiteren Ausführungsform des erfindungsgemäßen Lenkgetriebes mit Schneckenwellen,
- Fig. 8: eine perspektivische Detailansicht noch einer weiteren Ausführungsform des erfindungsgemäßen Lenkgetriebes für einen Linearantrieb mit Zahnstange,
- Fig. 9: eine Detailansicht nach Fig. 8 der Zahnstange,
- Fig. 10: eine perspektivische Ansicht der Betätigungseinheit mit einer weiteren Ausführungsform des erfindungsgemäßen Lenkgetriebes,
- Fig. 11: eine Teilschnittansicht des Lenkgetriebes nach Fig. 10,
- Fig. 12: eine Detailansicht auf einen Lenkring und ein Kraftübertrager nach Fig. 10 und 11, und
- Fig. 13: eine Schrittabfolge der Herstellung des Kraftübertragers nach Fig. 12.

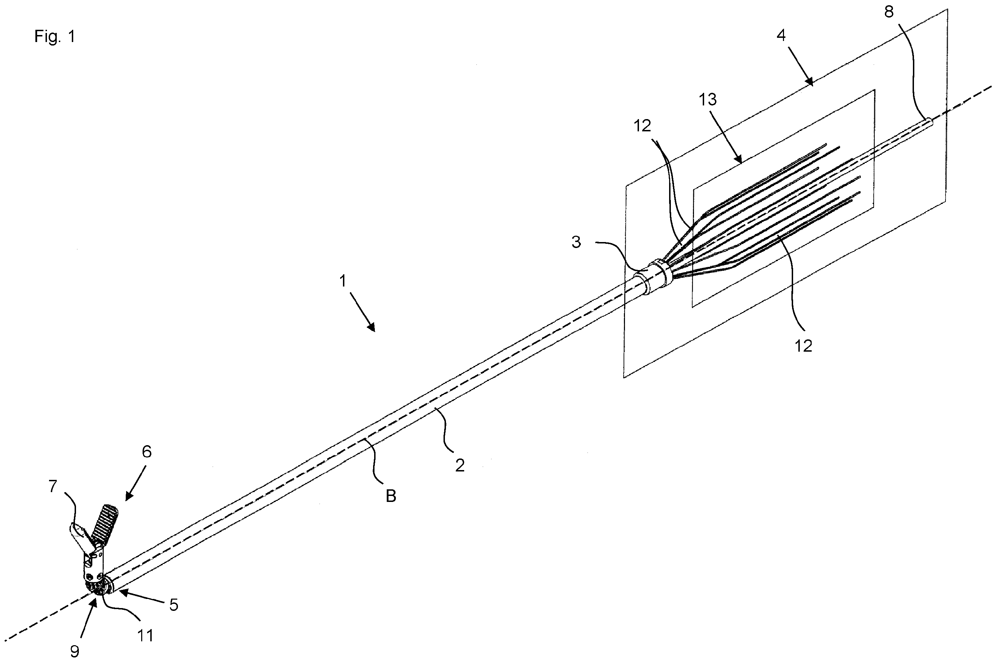

Fig. 1 zeigt schematisch ein chirurgisches Instrument 1 mit einem hohlen Schaft 2, einer am proximalen Ende 3 des Schaftes 2 angeordneten, nur schematisch dargestellten Betätigungseinheit 4 und einer am distalen Ende 5 des Schaftes 2 angeordneten Werkzeugspitze 6 mit einem Werkzeug 7. Das Werkzeug 7 ist über ein axial verschiebbar im Schaft 2 gelagertes Betätigungselement 8 betätigbar, das proximalseitig mit der Betätigungseinheit 4 in Wirkverbindung steht. Bei der Betätigungseinheit 4 kann es sich um eine manuell betätigbare Handhabe oder aber um eine für den robotischen Einsatz ausgelegte, also auch ohne manuelles Zutun betätigbare Baueinheit handeln - was vorteilhaft für die Reproduzierbarkeit der Betätigung ist. Bei dem Werkzeug 7 der Werkzeugspitze 6 kann es sich beispielsweise um ein mit Maulteilen versehenes Werkzeug, wie in Fig. 1 dargestellt, oder aber um ein Endoskop, einen Applikator oder dergleichen handeln. Die Werkzeugspitze 6 ist über einen Gelenkmechanismus 9 relativ zur Längsachse B des Schaftes 2 verschwenkbar, wobei der Gelenkmechanismus 9 aus am distalen Ende des Schaftes 5 angeordneten Schwenkgliedern 11 besteht, die über in Längsrichtung des Schaftes 2 verlaufende Lenkdrähte 12 so mit einem am proximalen Ende 3 des Schaftes 2 angeordneten Lenkgetriebe 13 verbunden sind, dass eine Bewegung des proximalseitigen Antriebs 13 eine entsprechende relative Bewegung der distalseitigen Schwenkglieder 11 und somit ein Verschwenken der Werkzeugspitze 6 verursacht. Auch wenn voranstehend und nachfolgend nur der Begriff Lenkdrähte 12 verwendet wird, können funktional auch Lenkseile verwendet werden, weshalb der verwendete Begriff Lenkdrähte 12 synonym auch als Lenkseil zu lesen und zu verstehen ist.

Das axial verschiebbar im Schaft 2 gelagerte Betätigungselement 8 zum Betätigen des beispielsweise aus zwei Maulteilen bestehenden Werkzeugs 7 ist bei der dargestellten Ausführungsform als Zug-/Schubstange ausgebildet.

Das Lenkgetriebe 13 für die Lenkdrähte 12 ist in dem chirurgischen Instrument 1 als motorisiertes Lenkgetriebe 13 ausgebildet.

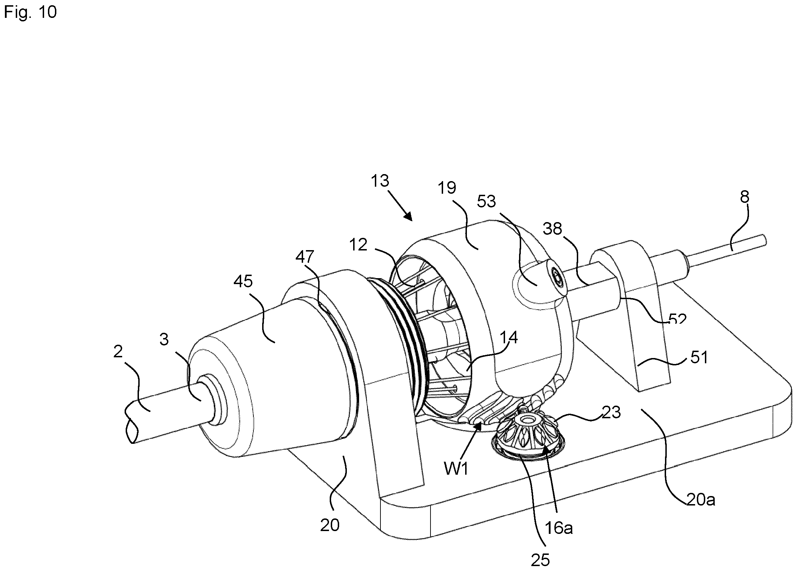

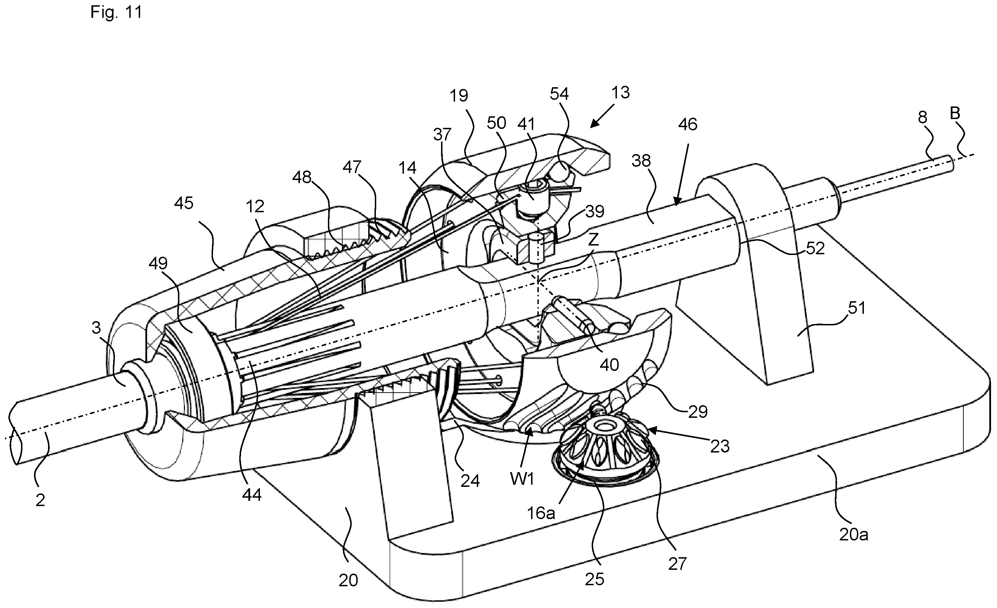

In Fig. 10 und 11 ist eine Ausgestaltung des Lenkgetriebes 13 mit einer räumlich verstellbaren Taumelscheibe 14 gezeigt, in der die Taumelscheibe 14 kardanisch auf einer koaxial zum Schaft 2 verlaufenden Hauptwelle 38 gelagert ist, um sie relativ zur Längsachse B des Schaftes 2 zu verlagern. Dabei sind die Lenkdrähte 12 so an der Taumelscheibe 14 gelagert bzw. befestigt, dass bei der Verlagerung der Taumelscheibe 14 über die Lenkdrähte 12 die Werkzeugspitze 6 verschwenkt wird.

Die Anzahl der zu verwendenden Lenkdrähte 12 für das motorisierte Lenkgetriebe 13 dort beträgt zehn, ohne dass ein erfindungsgemäßes Lenkgetriebe 13 darauf beschränkt sein soll. Die distalseitig parallel zur Längsachse B des Schaftes 2 verlaufenden Lenkdrähte 12 der Taumelscheibe 14 treten über ein Führungselement 44 aus dem Schaft 2 aus und werden der Taumelscheibe 14 unter Vergrößerung des Durchmessers des Lenkdrahtbündels zugeführt. Zum Festlegen der Lenkdrähte 12 an der Taumelscheibe 14 sind in dieser Durchgangsbohrungen 50 für jeden Lenkdraht 12 ausgebildet, die über radial in die Taumelscheibe 14 eingebrachte Madenschrauben 41 fixiert sind. Eine radiale Anordnung der Madenschrauben 41 macht eine Montage der Lenkdrähte 12 an der Taumelscheibe 14 erforderlich, bevor die Taumelscheibe 14 in dem Lenkring 19 angeordnet wird. Daher können die Madenschrauben anders als dargestellt abweichend von der radialen Richtung etwas geneigt oder verschoben angeordnet werden, sodass sie trotz Anordnung der Taumelscheibe im Lenkring zugänglich bleiben.

Alternative Befestigungsarten der Lenkdrähte an der Taumelscheibe sind denkbar, beispielsweise können die Lenkdrähte über eine proximalseitig hinter der Taumelscheibe angeordnete Klemmscheibe kraftschlüssig mit dieser verbunden sein oder einfach unlösbar durch Schweißen oder Kleben; figurativ nicht gezeigt.

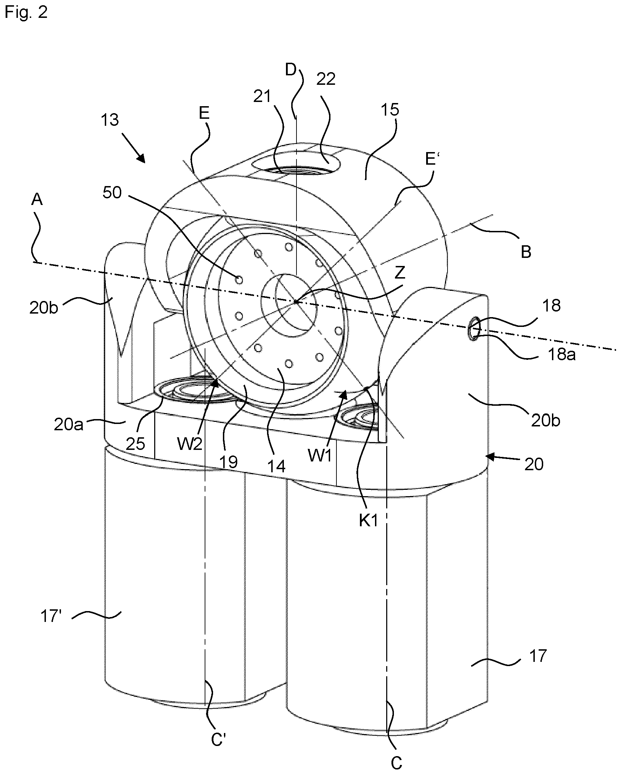

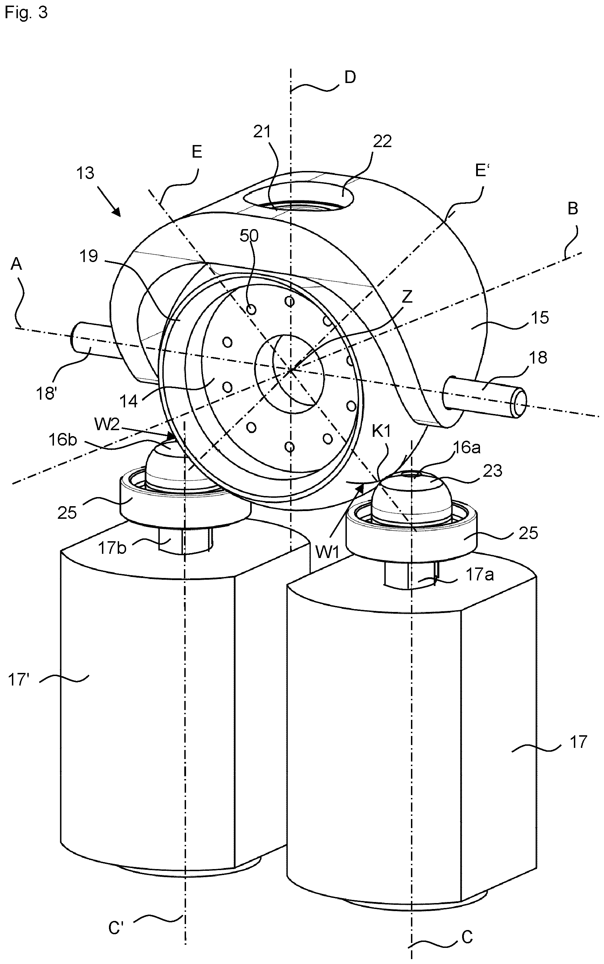

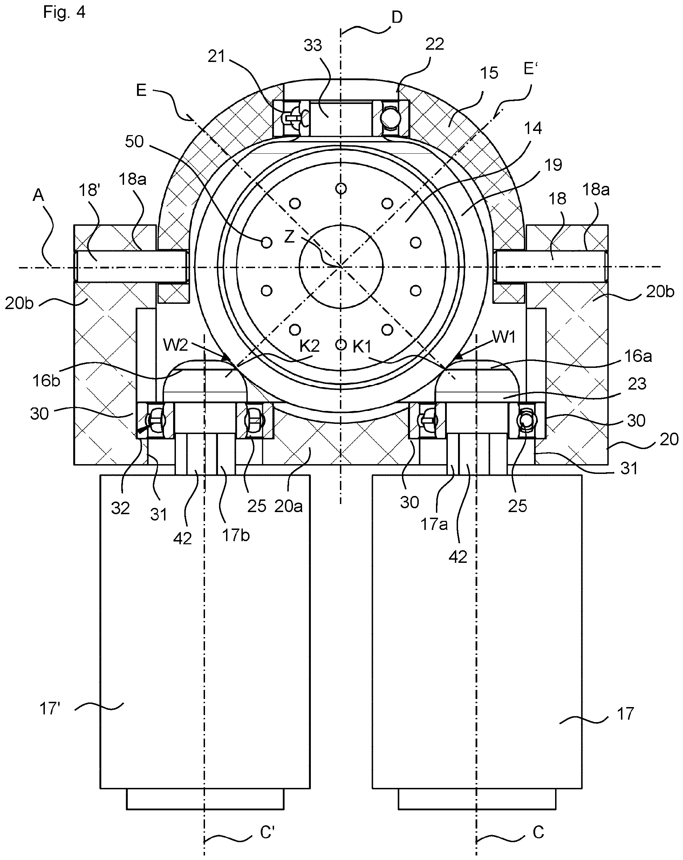

In Fig. 2 sind die zentralen Bauteile des Lenkgetriebes 13, die auch die Basis für die weiteren insbesondere für die in Fig. 3 bis 8 gezeigten Ausführungsformen sind, vereinfacht dargestellt: Das Gehäuse 20 hat eine Basis 20a und zwei sich gegenüberliegende seitliche Gehäuseteile 20b und ermöglicht die Lagerung des Bügels 15, der über Lagerstifte 18, 18' in entsprechenden Lagern bzw. Durchtrittsbohrungen 18a drehbar gelagert ist, wie in Fig. 4 ersichtlich. Damit ist der Bügel 15 um eine Schwenkachse A, die senkrecht zu der Längsachse B verläuft, schwenkbar. Zwischen den seitlichen Gehäuseteilen 20b ist die Taumelscheibe 14 angeordnet, die von einem Lenkring 19 entlang ihres Umfangs umfasst ist. Die Taumelscheibe 14 ist vorzugsweise drehbar im Lenkring 19 gelagert, beispielsweise wie in Fig. 11 zu sehen, mittels einer Wälz- bzw. dort Kugellagerung, damit die Taumelscheibe 14 mit der Hauptwelle 38 rotiert werden kann, während der Lenkring 19 nicht um die Längsachse B rotiert.

Der Lenkring 19 wird von dem Bügel 15 überspannt und wird in einer Durchgangsöffnung 22 des Bügels 15 mittels eines Lagerrings 21 drehbar um zur Schwenkachse A senkrecht verlaufende Drehachse D gehalten. Dazu hat der Lenkring 19 einen Achsstummel 33.

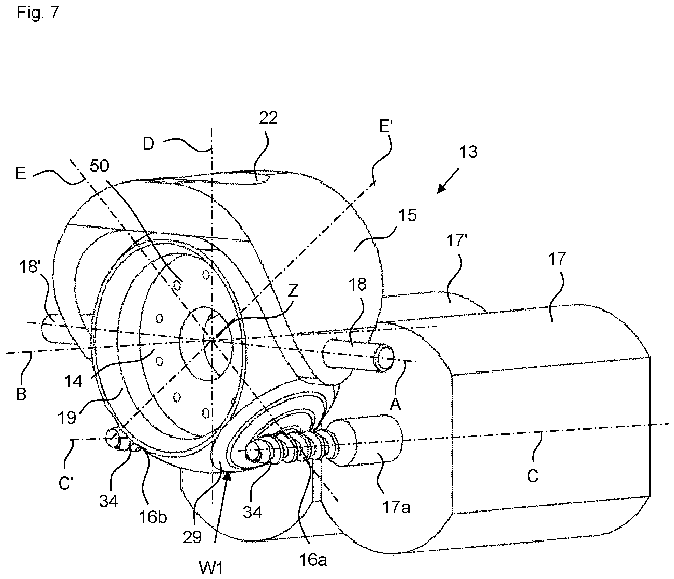

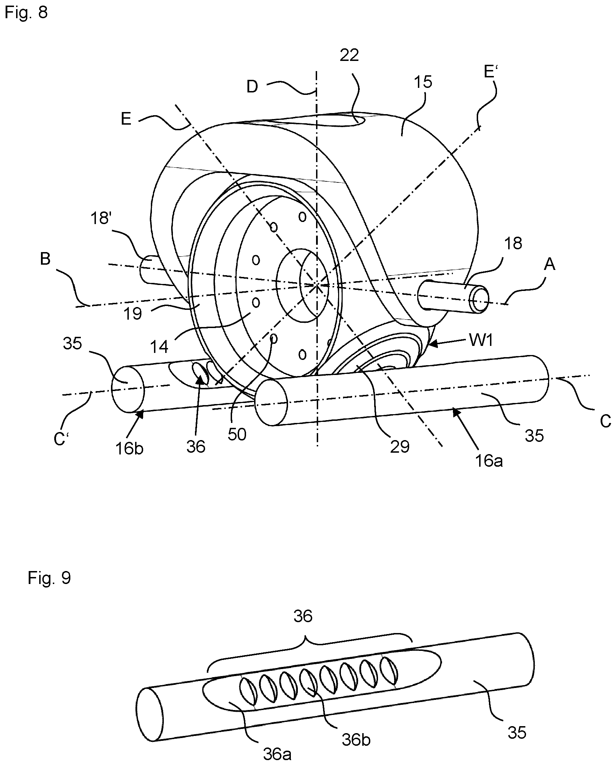

Unterhalb der Basis 20a sind in Fig. 2 bis 6 zwei Motoren 17, 17' angeordnet, deren Antriebsachsen C, C' parallel zueinander sind und senkrecht sowohl zu der Schwenkachse A als auch zu der Längsachse B verlaufen, ohne die Längsachse B zu schneiden. Ferner sind die Antriebsachsen C, C' in der gezeigten Darstellung des Lenkgetriebes 13 in neutraler Ausgangsstellung parallel zu der Drehachse D des Lenkringes 19, was sich allerdings je nach Verlagerung der Taumelscheibe 14 ändern kann. Im Beispiel von Fig. 7 und 8 verlaufen die Antriebsachsen C, C' ebenfalls parallel zueinander, aber auch parallel zur Längsachse B und senkrecht zur Schwenkachse A, allerdings ohne die Schwenkachse A zu schneiden. Die Motoren 17, 17' weisen Antriebswellen 17a, 17b auf, die in Fig. 3 bis 7 dargestellt sind. Die Antriebswellen 17a, 17b der Motoren 17, 17' ragen durch Durchgangsöffnungen mit jeweils zwei Öffnungsabschnitten 30, 31 in der Gehäusebasis 20a, wie insbesondere in Fig. 4 zu sehen ist.

In Fig. 3 bis 7 sind auf den Antriebswellen 17a, 17b Kraftübertrager 16a, 16b angeordnet, die die Rotationsbewegung der Antriebswellen 17a, 17b auf den Lenkring 19 zur Ausführung einer Dreh-/Kippbewegung übertragen. Jeder Kraftübertrager 16a, 16b ist mit dem Lenkring 19 gekoppelt, vorzugsweise in einem in den Figuren gezeigten unteren Bereich des Lenkrings 19, der einen jeweiligen Wirkabschnitt W1, W2 bildet. In dem Wirkabschnitt W1, W2 findet die direkte Kraftübertragung zwischen Kraftübertrager 16a, 16b und Lenkring 19 statt. Die Außenoberfläche des Lenkrings 19, dessen Form einer Kugelschicht bzw. Kugelscheibe entspricht, bildet dabei eine Kugelzone, wobei die beiden an einer entsprechenden Kugel gedachten parallelen Schnittebenen äquidistant bzw. symmetrisch zur Durchmesserebene verlaufen. Der Lenkring 19 hat damit in dem Wirkabschnitt W1, W2 eine teilsphärische Form, um seine Bewegungen zu koordinieren, und bei Verkippung den Kontakt mit den Kraftübertragern 16a, 16b zu erhalten, durch die die Bewegungen initiiert werden. Der Mittelpunkt der Kugel, auf der die Kugelzone basiert, die die Außenoberfläche des Lenkrings 19 darstellt, ist identisch mit einem Kreuzungspunkt der Drehachse D des Lenkrings 19 und der Schwenkachse A des Bügels 15, wobei dieser Kreuzungspunkt das kardanische Zentrum Z der kardanischen Aufhängung definiert.

Abweichend von den dargestellten Beispielen, kann der Lenkring auch nur in den Wirkabschnitten W1, W2 eine als Kugelkalotte geformte Außenoberfläche aufweisen, deren zugehöriges Kugelsegment auf demselben Kugelmittelpunkt basiert, der gleichzeitig das kardanische Zentrum Z ist.

Nachfolgend sind verschiedene Ausführungsformen für Kraftübertrager 16a, 16b und Wirkabschnitte W1, W2 des Lenkrings 19 beschrieben, wie in den Fig. 3 bis 9 gezeigt, wobei die Kraftübertrager 16a, 16b rotatorisch gelagerte Antriebskegel, gezeigt in Fig. 3 bis 6 und 10 bis 13, oder rotatorisch angetriebene Schneckenwellen, gezeigt in Fig. 7, oder auch linear angetriebene Antriebsstangen, dargestellt in Fig. 8 und 9, sein können.

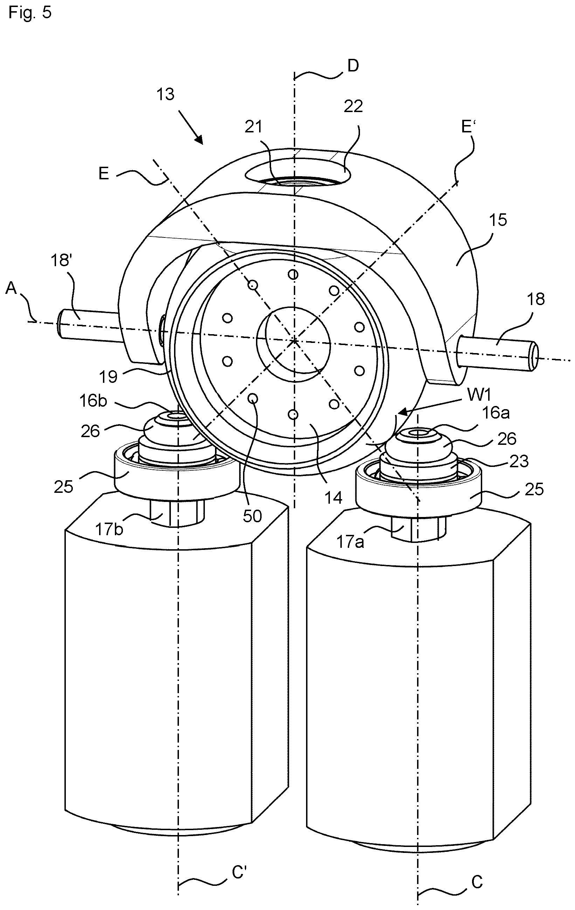

In Fig. 3 und 4 sind die Kraftübertrager 16a, 16b mit einer funktionellen Form zur Verdeutlichung des geometrischen Grundprinzips dargestellt. Die Kraftübertrager 16a, 16b sind rotatorisch gelagerte Antriebskegel mit einem Kegelkopf 23 und einem Kegelschaft 42. Als Kegelkopf 23 werden vorliegend nicht nur Kegelköpfe mit Kegel- oder Kegelstumpfform, sondern auch Köpfe 23 mit einer Kugelsegment- bzw.- Kugelscheiben-Form oder halbellipsoider Form verstanden, die ein gutes Verhältnis zwischen den Oberflächen von Kegelkopf 23 und Lenkring 19 bietet, wobei die jeweiligen Kontaktebenen an den Oberflächen von Kegelkopf 23 und Lenkring 19 in ihrem jeweiligen Kontaktpunkt K1, K2 jeweils senkrecht zur Achse E, E' zwischen diesem Kontaktpunkt K1, K2 und dem kardanischen Zentrum Z stehen. Die Form des Kegelkopfs 23 kann sich nach der Art der Wirkverbindung (reibschlüssig, formschlüssig) zwischen Lenkring 19 und Kegelkopf 23 richten. Ein Kegelkopf 23 mit Kugelsegment oder Kugelscheibenform mit kleinem Radius im Vergleich zu dem Radius des Lenkringes 19 kann beispielsweise für eine reibschlüssige Wirkverbindung bevorzugt sein, wie noch im Zusammenhang mit Fig. 5 beschrieben wird.

Beispielhaft an der Ausführungsform in Fig. 3 sei die Bewegungsübertragung und Taumelscheibenbewegung erläutert:
In den Kontaktpunkten K1, K2 zwischen Kraftübertrager 16a, 16b und Lenkring 19, wobei in Fig. 3 nur Kontaktpunkt K1 des ersten Kraftübertragers 16a mit dem Lenkring 19 zu sehen ist, ist die Drehbewegung des Kraftübertragers 16a, 16b bei infinitesimaler Betrachtung in diesem Punkt immer parallel zur Hauptachse B des chirurgischen Instruments 1 und in Bezug auf die Hauptachse B nach vorne (distal) oder hinten (proximal) gerichtet, je nach Drehrichtung. Diese Bewegung wird auf den Lenkring 19 übertragen. Durch dessen kardanische Aufhängung um das kardanische Zentrum Z ergibt sich so eine Verkippung des Lenkrings 19 um eine Kippachse E, E', die dem jeweiligen Antrieb (Motor 17, 17') zugeordnet ist.

Wird nun mittels des ersten Motors 17 die erste Antriebswelle 17a gedreht und mit ihr der erste Kraftübertrager 16a, so wird aufgrund der Wirkverbindung zwischen Lenkring 19 und dem ersten Kraftübertrager 16a die Drehbewegung des ersten Kraftübertragers 16a auf den ersten Wirkabschnitt W1 übertragen. Der zweite Kraftübertrager 16b bleibt währenddessen stehen, so dass der Lenkring 19, der im Wirkabschnitt W1 durch den ersten Kraftübertrager 16a bewegt wird, im Kontaktpunkt K2 des Wirkabschnitts W2 mit dem zweiten Kraftübertrager 16b stehen bleibt. Der Lenkring 19 wird damit verkippt und zwar um die zweite Kippachse E', die durch den Kontaktpunkt K2 im Wirkabschnitt W2 und das kardanische Zentrum Z führt und in einem Winkel von 45° zu sowohl der Drehachse D als auch der Drehachse A liegt. In diesem Fall, wenn der erste Kraftübertrager 16a sich von oben betrachtet gegen den Uhrzeigersinn dreht und der zweite Kraftübertrager 16b stillsteht, verkippt in Fig. 3 ein linker oberer Bereich des Lenkrings 19 um die zweite Kippachse E' nach hinten und gleichzeitig ein rechter unterer Bereich des Lenkrings 19 um die zweite Kippachse E' nach vorne. Umgekehrt bewirkt eine Drehung des zweiten Kraftübertragers 16b bei gleichzeitigem Stillstand des ersten Kraftübertragers 16b eine Kippung des Lenkrings 19 um die erste Kippachse E, die analog zu der zweiten Kippachse E' in einem Winkel von 45° zu sowohl der Drehachse D als auch der Drehachse A liegt und entsprechend durch den Kontaktpunkt K1 von erstem Kraftübertrager 16a und dem Wirkabschnitt W1 des Lenkrings 19 und durch das kardanische Zentrum Z führt, wo sich die beiden Kippachsen E, E' senkrecht schneiden. Drehen beide Kraftübertrager 16a, 16b, können zusammen alle Drehungen bzw. Verkippungen des Lenkrings 19 und damit der Taumelscheibe 14 im Raum bewirkt werden.

Dieses Prinzip ist auch die Basis für alle anderen hier dargestellten Ausführungsformen von Kraftübertragern 16a, 16b.

Um den Kegelschaft 42 jedes Antriebskegels ist zu dem Kegelkopf 23 benachbart ein Lagerring 25 (vgl. Fig. 2 bis 6) angeordnet, der vorzugsweise ein Wälzlager, beispielsweise ein Rollen- oder ein Kugellager haust. Der Lagerring 25 sorgt für eine geführte Rotation der Antriebskegel und damit eine gute Kraftübertragung der Rotationsbewegung auf den Lenkring 19. Um den jeweiligen Lagerring 25 zu halten, weist das Gehäuse 20 in seiner Basis 20a zwei Durchtrittsöffnungen für die Antriebswellen 17a, 17b mit jeweils zwei Öffnungsabschnitten 30, 31 auf, wie insbesondere in Fig. 4 zu sehen ist. Die Öffnungsabschnitte 30, 31 sind miteinander verbunden und haben einen unterschiedlichen Durchmesser, wobei die Öffnungsabschnitte 31, die zu ihrem jeweiligen Motor 17, 17' gerichtet sind, einen kleineren Durchmesser haben als die darüber liegenden Öffnungsabschnitte 30, die zu dem Lenkring 19 gerichtet sind. Hierdurch wird an der Verbindungsstelle beider Öffnungsabschnitte 30, 31 eine Schulter 32 gebildet, wobei jeder der Lagerringe 25 auf der jeweiligen Schulter 32 aufliegt, wie in Fig. 4 dargestellt.

In Fig. 5 sind die Kraftübertrager 16a, 16b wie in Fig. 3 und 4 rotatorisch gelagerte Antriebskegel mit Kegelkopf 23, der hierbei für eine reibschlüssige Wirkverbindung mit einem Reibelement ausgebildet ist. Das gezeigte Reibelement ist ein um den Kegelkopf 23 umfänglich angelegtes toroidales Reibband 26, wie bspw. ein Gummiband oder Gummiring, sodass hier die Wirkverbindung zwischen Kraftübertragern 16a, 16b und Lenkring 19 mittels Reibungskraft hergestellt wird. Das Reibelement kann alternativ ein am Kegelkopf 23 umfänglich aufgebrachtes reibungserhöhendes Material sein. Dazu, aber auch für einen verbesserten Halt eines umfänglich angelegten Reibbands 26 mittels Formschluss kann eine um den Kegelkopf 23 umlaufenden Nut vorgesehen sein, in die das reibungserhöhende Material beim Aufbringen eindringt bzw. ein entsprechend ausgeformter innenumfänglicher Abschnitt des Reibbands 26 aufgenommen wird. Das für das Reibelement verwendete Material stellt vorzugsweise mit dem Lenkring eine Reibkraft bereit, die höher ist als die Reibkraft, die zwischen dem Material des Kegelkopfs 23 und dem Lenkring 19 besteht. Durch die hohe Reibkraft zwischen den Bauteilen Kegelkopf 23 und Wirkabschnitt W1, W2 des Lenkrings 19 wird die Wirkverbindung durch Reibung bereitgestellt.

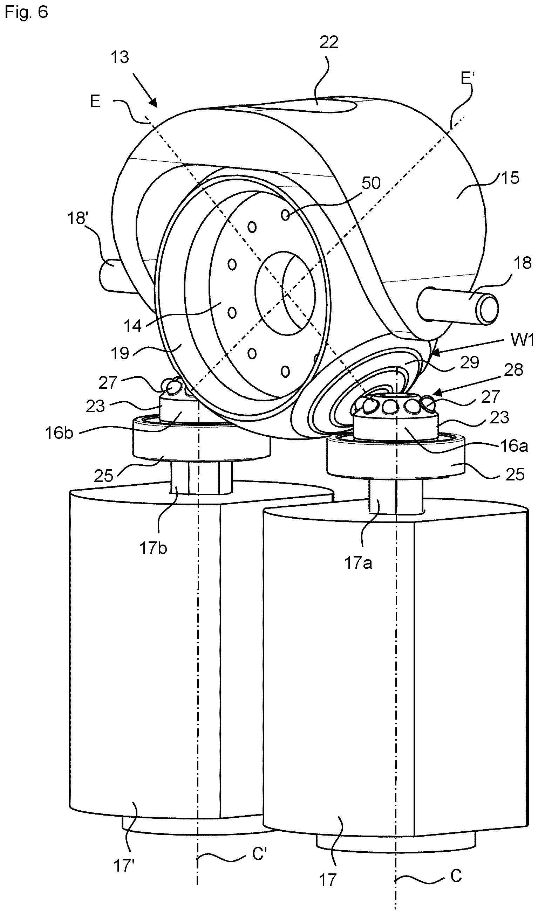

In Fig. 6 wird die Wirkverbindung zwischen Kraftübertragern 16a, 16b und Lenkring 19 durch einen formschlüssigen Eingriff gebildet. Erneut sind der erste und der zweite Kraftübertrager 16a, 16b rotatorisch gelagerte Antriebskegel mit einem Kegelkopf 23. In dieser Ausführungsform sind an dem jeweiligen Kegelkopf 23 umfänglich eine Vielzahl von Antriebsnoppen 27 angeordnet, die einen umlaufenden Noppenkranz 28 bilden. Damit die Noppen 27 mit dem Lenkring 19 in Eingriff kommen können, weist der Lenkring 19 an seinem Wirkabschnitt W1 und entsprechend auch dem zweiten Wirkabschnitt, der in Fig. 6 nicht zu sehen ist, Ausnehmungen 29 auf, die mit den Antriebsnoppen 27 der beiden Noppenkränze 28 kämmen. Um eine Bewegungsübertragung zu erreichen, sind die Ausnehmungen 29 als konzentrische Ringe mit einem an die Größe und den Zahnungsabstand der Noppen 27 angepassten Durchmesser und mit entsprechender Breite in dem Wirkabschnitt W1 ausgebildet.

In Fig. 7 wird die Wirkverbindung zwischen Kraftübertragern 16a, 16b und Lenkring 19 ebenfalls durch einen formschlüssigen Eingriff gebildet, wobei der erste und der zweite Kraftübertrager 16a, 16b Schneckenwellen 34 sind. Diese Schneckenwellen 34 verlaufen parallel zu der Längsachse B, so dass die Antriebsachsen C, C' der Motoren 17, 17' ebenfalls parallel zur Hauptachse B verlaufen. Wie schon in der Ausführungsform in Fig. 6 der Fall, weist der Lenkring 19 an seinem Wirkabschnitt W1 und entsprechend auch dem zweiten Wirkabschnitt, der in Fig. 7 nicht zu sehen ist, konzentrisch angeordnete Ausnehmungen 29 auf, die mit der Schneckenwelle 34 kämmen, wenn die Schneckenwelle 34 sich dreht. Die Bewegungsübertragung ist ebenfalls direkt und entspricht dem Ablauf der Bewegungsübertragung wie auch schon zu den rotatorisch angetriebenen Antriebskegeln erläutert wurde.

Fig. 8 und 9 zeigen eine alternative Antriebsart: Anstatt eines rotatorischen Antriebselement sind der erste und der zweite Kraftübertrager 16a, 16b Zahnstangen 35. Diese sind mit einem Zahnungsabschnitt 36 ausgestattet. Der Lenkring 19 weist hier analog zu den vorherigen Ausführungsformen an seinem Wirkabschnitt W1 und entsprechend auch dem zweiten Wirkabschnitt, der in Fig. 8 nicht zu sehen ist, konzentrisch angeordnete Ausnehmungen 29 auf, die mit den Zahnungsabschnitten 36 der beiden Zahnstangen 35 kämmen können. Die hier gebildete Wirkverbindung ist ausgebildet für einen Linearantrieb, dessen Antriebselement (figurativ nicht dargestellt), das in einem erfindungsgemäßen Lenkgetriebe der Antriebswelle entsprechen soll, die die Antriebsachse C, C' definiert, nicht rotiert, sondern parallel zur Hauptachse B vor- und zurückbewegt wird. Der Zahnungsabschnitt besteht aus einer länglichen Ausnehmung 36a und in dieser Ausnehmung angeordnete, herausgearbeitete Zähne 36b, die linear und äquidistant voneinander beabstandet sind.

In Fig. 10 und 11 ist eine alternative kardanische Lagerung des Lenkrings 19 dargestellt: Der Lenkring 19 umfasst die Taumelscheibe 14, die auf einer Kreuzgelenkscheibe 37 angeordnet ist, die auf der Hauptwelle 38 gelagert ist. Die Taumelscheibe 14 ist hierzu über zwei um 180° versetzt zueinander, d. h. koaxial und diametral angeordnete Lagerstifte 40 verschwenkbar auf einer Kreuzgelenkscheibe 37 gelagert, die wiederrum über zwei um 180° versetzt zueinander, d. h. koaxial und diametral angeordnete Lagerstifte 39 verschwenkbar auf der Hauptwelle 38 gelagert ist. Aus Gründen der besseren Übersicht ist in Fig. 11 jeweils nur ein Lagerstift 39 und ein Lagerstift 40 dargestellt. Die Lagerstifte 40 der Taumelscheibe 14 und die Lagerstifte 39 der Kreuzgelenkscheibe 37 sind um 90° versetzt zueinander angeordnet, wobei die Stiftachsen das kardanischen Zentrum Z definieren. Diese Lagerung ermöglicht es, die Taumelscheibe 14 um zwei rechtwinklig zueinanderstehende Achsen relativ zur Längsachse B des Schaftes 2 zu verschwenken, wodurch über die Lenkdrähte 12 distalseitig die Werkzeugspitze 6 (vgl. Fig. 1) in alle Raumrichtungen relativ zur Längsachse B des Schaftes 2 verschwenkt werden kann. Ein Bügel, wie er in den Fig. 2 bis 8 vorgesehen war, ist bei dieser inneren kardanischen Lagerung der Taumelscheibe 14 nicht notwendig. Damit der Lenkring 19 sich auch bei dieser inneren kardanischen Lagerung ausschließlich um die Quer- und Hochachse inklusive Überlagerungen drehen kann und seine Position im Raum fixiert und eine Drehung um die Hauptachse B unterbunden wird, ist hier der Lenkring 19 in Bezug auf die Längsachse B drehfest mit dem Gehäuse 20 gekoppelt, indem ein sich von dem Lenkring 19 an der Unterseite radial nach außen erstreckender Stift (nicht zu sehen), in einer Nut 24 des Gehäuses 20 (siehe Fig. 11) geführt wird, die sich parallel zur Längsachse B entlang dem Bewegungsradius des Stifts erstreckt.

Die Hauptwelle 38 ist an ihrem distalen Ende 3, das dem proximalen Ende 3 des Schafts 2 entspricht, von einer Schutzkappe 45 umgeben, die den Eintritt des Schafts 2 und damit der Hauptwelle 38 in das Gehäuse 20 schützt. Die Schutzkappe 45 ist mit einem Außengewinde 47 in ein in eine Gehäuseöffnung mit entsprechendem Gewinde 48 eingeschraubt. Die Schutzkappe 45 umhüllt distalseitig ferner ein Kugellager 49, in dem die Hauptwelle 38 drehbar gelagert ist. Benachbart zu diesem Kugellager 49 ist das Führungselement 44 um die Hauptwelle 38 angeordnet, um die Lenkdrähte 12 aus dem Schaft 2 austreten zu lassen und der Taumelscheibe 14 zuzuführen, wobei sich der Durchmesser des Lenkdrahtbündels entsprechend aufweitet.

Proximalseitig hinter dem Führungselement 44 werden die zur Längsachse B des Schaftes 2 stetig aufweitend verlaufenden Lenkdrähte 12 der Taumelscheibe 14 zugeführt. Zum Festlegen der Lenkdrähte 12 an der Taumelscheibe 14 sind in der Taumelscheibe 14 Durchgangsöffnungen 50 für jeden Lenkdraht 12 ausgebildet, wobei im dargestellten Beispiel die Lenkdrähte 12 innerhalb der Durchgangsbohrungen 50 über Madenschrauben 41 kraftschlüssig mit der Taumelscheibe 14 verbunden und fixiert sind.

Die Hauptwelle 38 ist an ihrem proximalen Ende 46 in einem weiteren Gehäuseteil 51 in einer Durchtrittöffnung 52 etwa mittels eines Gleitlagers oder eines Wälzlagers drehbar gelagert. Weiter proximalseitig des Gehäuseteils 51 kann die Betätigungseinheit 4 einen nicht dargestellten Antrieb zur Bewegung des Betätigungselements 8 aufweisen.

Der Lenkring 19, der in dem Lenkgetriebe 13 nach Fig. 10 und 11 eingesetzt ist, benötigt keine separate Aufhängung durch einen Bügel, da die kardanische Aufhängung und Lagerung auf der Hauptwelle 38 erfolgt. Damit die Taumelscheibe 14 mit der Hauptwelle 38 um die Längsachse B rotieren kann, ist die Taumelscheibe 14 durch eine Wälzlagerung drehbar in dem Lenkring 19 gelagert. In Fig. 11 ist eine Kugel als Wälzkörper 54 zu sehen, wobei das Wälzlager vorzugsweise als vollkugeliges Lager ausgeführt ist. Im gezeigten Beispiel sind ferner die Lagerlaufflächen nicht in separaten Lagerschalen, sondern direkt an der äußeren Mantelfläche der Taumelscheibe 14 und an der inneren Mantelfläche des Lenkrings 19 ausgebildet, weshalb der Lenkring 19 einen Füllstutzen 53 zum Einfüllen der Wälzkörper-Kugeln 54 aufweist. Der Verzicht auf Lagerschalen ermöglicht einen kompakteren Aufbau der Taumelscheiben-Lenkring-Kombination. Nach der Befüllung des Wälzlagers wird der Füllstutzen 53 beispielsweise mit einer Schraube als Stopfen geschlossen.

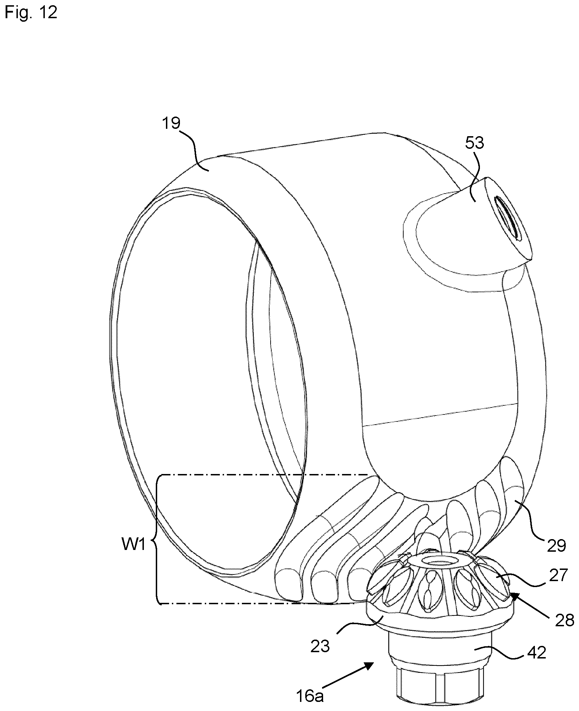

Der Lenkring 19 wird mit einem rotatorisch angetriebenen Antriebskegel als Kraftübertrager 16a bewegt, dessen Kegelkopf 23, wie auch insbesondere in Fig. 11 und 12 gezeigt ist, durch die Durchgangsöffnung in der Gehäusebasis 20a ragt und durch den Lagerring 25 in seiner Rotation geführt und gelagert ist. Der Kegelkopf 23 weist hier einen Noppenkranz 28 auf, der halbellipsoide Noppen 27 trägt. In Fig. 11 und 12 ist der Kegelkopf 23, der auf dem Kegelschaft 42 ruht, im Eingriff mit dem Wirkabschnitt W1 des Lenkrings 19 und kämmt mit seinem Noppenkranz 28 den Wirkabschnitt W1 des Lenkrings 19. Der Wirkabschnitt W1 des Lenkrings 19 weist bogenförmige Ausnehmungen 29 auf, die der ellipsoiden Form der Noppen 27 des Kegelkopfes 23 folgen.

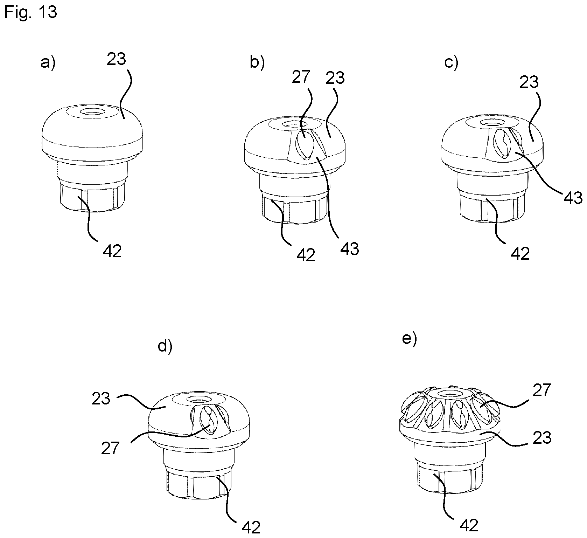

In Fig. 13 a) bis e) ist die zerspanende Herstellung des in Fig. 11 und 12 gezeigten Kegelkopfes 23 schrittweise abgebildet. Es wird in einem ersten Schritt nach Fig. 13 a) von einem pilzförmigen Antriebskegel mit halbellipsoidem Kegelkopf 23 auf dem Schaft 42 ausgegangen. In den Mantel des Kegelkopfes 23 wird eine Ausnehmung 43 eingebracht und eine ellipsoide Noppe 27 stehen gelassen (Fig. 13 b)). In zwei weiteren Schritten nach Fig. 13 c) und d) werden die Noppen 27 in ihre halbellipsoide Form gebracht und facettiert. Benachbart werden dann die restlichen Noppen 27 auf die gleiche Weise erzeugt. Fig. 13 e) zeigt den Endzustand, wenn alle Noppen 27 in der halbellipsoiden Form geformt sind und er Noppenkranz 28 vollständig ausgebildet ist. Alternativ dazu und für größere Stückzahlen bevorzugt kann ein derart geformter Antriebskegel im Spritzguss hergestellt werden.

Ein wie zuvor beschrieben ausgebildetes chirurgisches Instrument 1 zeichnet sich dadurch aus, dass viele dünne Lenkdrähte 12 zur Ansteuerung der verschwenkbaren Werkzeugspitze 6 verwendet werden können, und diese Ansteuerung auf Grund des motorisch angetriebenen Lenkgetriebes 13 für die Taumelscheibe 14, an der die Lenkdrähte 12 gelagert sind, feinfühlig, exakt und reproduzierbar erfolgt.

Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen. Die vorliegende Erfindung stellt ein Lenkgetriebe 13 für ein chirurgisches Instrument 1 bereit, wobei das Lenkgetriebe 13 am proximalen Ende 3 eines Schafts 2 anordenbar ist, mit einer Längsachse B und einer Abwinklungsmechanik 9 am distalen Ende 5. Das Lenkgetriebe 13 weist zwei motorisierte Antriebe auf und richtet über die Stellwinkel der zwei Antriebe eine Taumelscheibe 14 räumlich aus, um die Abwinkelungsmechanik 9 des chirurgischen Instruments 1 zu steuern. Die Taumelscheibe 14 ist in einem Lenkring 19 angeordnet, wobei die beiden motorisierten Antriebe jeweils eine von einem Motor 17, 17' angetriebene Antriebswelle 17a, 17b aufweisen, die mit dem Lenkring 19 jeweils über einen Kraftübertrager 16a, 16b direkt in Wirkverbindung stehen. Der erste Kraftübertrager 16a kontaktiert den Lenkring 19 an einem ersten Wirkabschnitt W1, und zweite Kraftübertrager 16b kontaktiert den Lenkring 19 an einem zweiten Wirkabschnitt W2, wobei der Lenkring 19 um ein kardanisches Zentrum Z kardanisch gelagert ist und zumindest an dem ersten und dem zweiten Wirkabschnitt W1, W2 kugelsegmentförmig ausgebildet ist, wobei die Wirkabschnitte W1, W2 einen gemeinsamen Kugelmittelpunkt aufweisen, in dem sich eine erste Kippachse E, die durch einen Kontaktpunkt K1 des ersten Kraftübertragers 16a mit dem Lenkring 19 und das kardanische Zentrum Z definiert wird, und eine zweite Kippachse E', die durch einen Kontaktpunkt K2 des zweiten Kraftübertragers 16b mit dem Lenkring 19 und das kardanische Zentrum Z definiert wird, im rechten Winkel schneiden. Ferner wird ein chirurgisches Instrument 1 offenbart, das ein solches Lenkgetriebe 13 aufweist.

### BEZUGSZEICHENLISTE

- 1: Chirurgisches Instrument
- 2: Schaft
- 3: Proximales Ende (Schaft)
- 4: Betätigungseinheit
- 5: Distales Ende (Schaft)
- 6: Werkzeugspitze
- 7: Werkzeug
- 8: Betätigungselement
- 9: Gelenkmechanismus
- 11: Schwenkglied
- 12: Lenkdraht
- 13: Lenkgetriebe
- 14: Taumelscheibe (räumlich verstellbar)
- 15: Bügel
- 16a, 16b: Kraftübertrager
- 17, 17': Motor
- 17a, 17b: Antriebswelle
- 18, 18': Lagerstifte
- 18a: Durchgangsbohrung
- 19: Lenkring
- 20: Gehäuse
- 20a: Basis
- 20b: Seitliche Gehäuseteile
- 21: Kugellager für Lenkring
- 22: Aufnahmeöffnung
- 23: Antriebskegelkopf
- 24: Führungsnut
- 25: Lagerring
- 26: Reibband
- 27: Noppen
- 28: Noppenkranz
- 29: Ausnehmungen
- 30: Erster Abschnitt Durchtrittsöffnung
- 31: Zweiter Abschnitt Durchtrittsöffnung
- 32: Schulter
- 33: Achsstummel
- 34: Schneckenwelle
- 35: Zahnstange
- 36: Zahnungsabschnitt
- 36a: Ausnehmung
- 36b: Zähne
- 37: Kreuzgelenkscheibe
- 38: Hauptwelle
- 39: Lagerstift
- 40: Lagerstift
- 41: Madenschraube
- 42: Kegelschaft
- 43: Ausnehmung
- 44: Führungselement
- 45: Schutzkappe
- 46: proximales Ende Hauptwelle 38
- 47: Außengewinde
- 48: Gewinde in Gehäuseöffnung
- 49: Kugellager
- 50: Durchgangsöffnungen Taumelscheibe 14
- 51: Gehäuseteil
- 52: Durchtrittöffnung
- 53: Füllstutzen
- 54: Wälzkörper

- A: Schwenkachse Befestigungsvorrichtung Bügel 15
- B: Längsachse des Instruments bzw. des Schafts
- C, C: Antriebsachse des ersten Antriebs 17', des zweiten Antriebs
- D: Drehachse Lenkring
- E, E': Erste, zweite Kippachse Lenkring
- K1, K2: Kontaktpunkt erster, zweiter Kraftübertrager-Lenkring
- W1, W2: Erster, zweiter Wirkabschnitt
- Z: Kardanisches Zentrum

## Patentansprüche

1. Lenkgetriebe (13) für ein chirurgisches Instrument (1), wobei das Lenkgetriebe (13) eine Taumelscheibe (14) aufweist, wobei das Lenkgetriebe (13)
am proximalen Ende (3) eines Schafts (2) anordenbar ist, der eine Längsachse (B) definiert und am distalen Ende (5) eine Abwinkelungsmechanik (9) aufweist,
wobei das Lenkgetriebe (13) zwei motorisierte Antriebe aufweist und dazu ausgebildet ist, über die Stellwinkel der zwei Antriebe die Taumelscheibe (14) räumlich auszurichten, die dazu ausgebildet ist, die distale Abwinkelungsmechanik (9) des chirurgischen Instruments (1) zu steuern,
wobei die Taumelscheibe (14) in einem Lenkring (19) angeordnet ist, **dadurch gekennzeichnet, dass** der erste der zwei motorisierten Antriebe eine von einem ersten Motor (17) angetriebene erste Antriebswelle (17a) aufweist, die mit dem Lenkring (19) über einen ersten Kraftübertrager (16a) direkt in Wirkverbindung steht, indem der erste Kraftübertrager (16a) unmittelbar den Lenkring (19) an einem ersten Wirkabschnitt (W1) kontaktiert, wobei der erste Kraftübertrager (16) auf der ersten Antriebswelle (17a) angeordnet ist, die eine erste Antriebssachse (C) definiert, und
der zweite der zwei motorisierten Antriebe eine von einem zweiten Motor (17') angetriebene zweite Antriebswelle (17b) aufweist, die mit dem Lenkring (19) über einen zweiten Kraftübertrager (16b) direkt in Wirkverbindung steht, indem der zweite Kraftübertrager (16b) unmittelbar den Lenkring (19) an einem zweiten Wirkabschnitt (W2) kontaktiert, wobei der zweite Kraftübertrager (16b) auf der zweiten Antriebswelle (17b) angeordnet ist, die eine zweite Antriebsachse (C') definiert,
wobei der Lenkring (19) um ein kardanisches Zentrum (Z) kardanisch gelagert ist und zumindest an dem ersten und dem zweiten Wirkabschnitt (W1, W2) kugelsegmentförmig ausgebildet ist, wobei die kugelsegmentförmigen Wirkabschnitte (W1, W2) einen gemeinsamen Kugelmittelpunkt aufweisen, der dem kardanischen Zentrum (Z) entspricht, in dem sich eine erste Kippachse (E), die durch einen Kontaktpunkt (K1) des ersten Kraftübertragers (16a) mit dem Lenkring (19) und das kardanische Zentrum (Z) definiert wird, und eine zweite Kippachse (E'), die durch einen Kontaktpunkt (K2) des zweiten Kraftübertragers (16b) mit dem Lenkring (19) und das kardanische Zentrum (Z) definiert wird, im rechten Winkel schneiden.

2. Lenkgetriebe (13) nach Anspruch 1,
**dadurch gekennzeichnet, dass**
die kardanische Lagerung des Lenkrings (19) bereitgestellt wird, indem
a) der Lenkring (19) an einer ersten Befestigungsvorrichtung kardanisch aufgehängt ist, wobei die erste Befestigungsvorrichtung ein Bügel (15) ist, der
- an einem Abschnitt des Lenkrings (19) angeordnet ist, der von den Wirkabschnitten (W1, W2) abgewandt ist, und
- beidenends an einem Gehäuse (20) drehbar um eine Schwenkachse (A) gelagert ist, die senkrecht zu der Längsachse (B) und senkrecht zu den Antriebsachsen (C, C') verläuft, und
- mittig eine Aufnahmeöffnung (22) aufweist,
wobei der Lenkring (19) in der Aufnahmeöffnung (22) drehbar um eine Drehachse (D) gelagert ist, die senkrecht zu der Schwenkachse (A) verläuft;
oder indem
b) der Lenkring (19) über die Taumelscheibe (14), die um die Längsachse (B) rotierbar in dem Lenkring (30) gelagert oder fest mit dem Lenkring (19) verbunden ist, kardanisch auf einer koaxial zu der Längsachse (B) des Schaftes (2) verlaufenden Hauptwelle (38) gelagert ist, wobei die Taumelscheibe (14) über zwei um 180° versetzt zueinander angeordnete Lagerstifte (39) verschwenkbar auf einer Kreuzgelenkscheibe (37) gelagert ist, und die Kreuzgelenkscheibe (28) über zwei um 180° versetzt zueinander angeordnete Lagerstifte (40) verschwenkbar auf der Hauptwelle (38) gelagert ist, und wobei die Lagerstifte (39, 40) der Taumelscheibe (14) und der Kreuzgelenkscheibe (37) um 90° versetzt zueinander angeordnet sind.

3. Lenkgetriebe (13) nach Anspruch 1 oder 2,
**dadurch gekennzeichnet, dass**
die Wirkverbindung einen formschlüssigen Eingriff bereitstellt, indem
- der erste und der zweite Kraftübertrager (16a, 16b) rotatorisch gelagerte Antriebskegel mit einem Kegelkopf (23) sind, der umfänglich eine Vielzahl von Antriebsnoppen (27) einen Noppenkranz (28) bildend aufweist, und wobei der Lenkring (19) an jedem Wirkabschnitt (W1, W2) Ausnehmungen (29) aufweist, die mit den Antriebsnoppen (27) der beiden Noppenkränze (28) kämmen, oder
- der erste und der zweite Kraftübertrager (16a, 16b) Schneckenwellen (34) sind, die parallel zu der Längsachse (B) verlaufen, und wobei der Lenkring (19) an jedem Wirkabschnitt (W1, W2) konzentrisch angeordnete Ausnehmungen (29) aufweist, die mit der Schneckenwelle (34) kämmen.

4. Lenkgetriebe (13) nach Anspruch 3,
**dadurch gekennzeichnet, dass**
die Antriebsnoppen (27) eine halbkugelige oder eine halbellipsoide Form haben, und/oder
die Ausnehmungen (29) konzentrisch ineinander angeordnete Ringe oder bogenförmige Nuten sind.

5. Lenkgetriebe (13) nach Anspruch 1 oder 2,
**dadurch gekennzeichnet, dass**
die Wirkverbindung durch Reibung bereitgestellt wird, indem
- der erste und der zweite Kraftübertrager (16a, 16b) rotatorisch gelagerte Antriebskegel mit einem Kegelkopf (23) sind, der umfänglich ein Reibelement aufweist, bevorzugt ein um den Kegelkopf (23) aufgebrachtes Material, das eine höhere Reibung bereitstellt als das Material, aus dem der Kegelkopf (23) besteht, oder ein um den Kegelkopf (23) umfänglich angelegtes Reibband (26), das ein Gummiband oder ein Band mit aufgerauter Oberfläche oder ein Band mit einer Zahnung oder Noppen ist.

6. Lenkgetriebe (13) nach Anspruch 1 oder 2,
**dadurch gekennzeichnet, dass**
die Wirkverbindung des ersten Kraftübertragers (16a) mit dem Lenkring (19) einen formschlüssigen Eingriff bereitstellt und die Wirkverbindung des zweiten Kraftübertragers (16b) mit dem Lenkring (19) durch reibschlüssigen Eingriff bereitgestellt wird,
wobei der formschlüssige Eingriff des ersten Kraftübertragers (16a) mit dem Lenkring (19) bereitgestellt wird, indem der erste Kraftübertrager (16a)
- ein rotatorisch gelagerter Antriebskegel mit einem Kegelkopf (23) ist, der umfänglich eine Vielzahl von Antriebsnoppen (27) einen Noppenkranz (28) bildend aufweist, wobei der Lenkring (19) an dem ersten Wirkabschnitt (W1) Ausnehmungen (29) aufweist, die mit den Antriebsnoppen (27) der beiden Noppenkränze (28) kämmen, oder
- eine Schneckenwelle (34) ist, die parallel zu der Längsachse (B) verläuft, und wobei der Lenkring (19) an dem ersten Wirkabschnitt (W1) konzentrisch angeordnete Ausnehmungen (29) aufweist, die mit der Schneckenwelle (34) kämmen,
und wobei der reibschlüssige Eingriff des zweiten Kraftübertragers (16b) mit dem Lenkring (19) bereitgestellt wird, indem
der zweite Kraftübertrager (16b) ein rotatorisch gelagerter Antriebskegel mit einem Kegelkopf (23) ist, der umfänglich ein Reibelement aufweist, das bevorzugt
- ein um den Kegelkopf (23) aufgebrachtes Material ist, das eine höhere Reibung bereitstellt als das Material, aus dem der Kegelkopf (23) besteht, oder
- ein um den Kegelkopf (23) umfänglich angelegtes Reibband (26) ist, das ein Gummiband oder ein Band mit aufgerauter Oberfläche oder ein Band mit einer Zahnung oder Noppen ist.

7. Lenkgetriebe (13) nach zumindest einem der Ansprüche 3 bis 6,
**dadurch gekennzeichnet, dass**
die rotatorisch gelagerten Antriebskegel jeweils einen Kegelschaft (42) haben, um den benachbart zu dem Kegelkopf (23) ein Lagerring (25) angeordnet ist, und dass das Gehäuse (20) eine Basis (20a) mit jeweils einer Durchtrittsöffnung für jede Antriebsachse (17a, 17b) aufweist, wobei jede Durchtrittsöffnung einen ersten Öffnungsabschnitt (30) mit einem Durchmesser, der einem Durchmesser des Lagerrings (25) entspricht, und einen zum ersten Öffnungsabschnitt (30) koaxialen zweiten Öffnungsabschnitt (31) mit einem Durchmesser aufweist, der kleiner als der Durchmesser des ersten Öffnungsabschnitts (30) ist, sodass das Gehäuse (20) in jeder Durchtrittsöffnung eine Schulter (32) bildet, auf der der jeweilige Lagerring (25) aufliegt.

8. Lenkgetriebe (13) nach Anspruch 1 oder 2,
**dadurch gekennzeichnet, dass**
die Wirkverbindung
- einen Linearantrieb bildet, indem der erste und der zweite Kraftübertrager (16a, 16b) Zahnstangen (35) mit einem Zahnungsabschnitt (36) sind und wobei der Lenkring (19) an seinem Wirkabschnitt (W) konzentrisch angeordnete Ausnehmungen (29) aufweist, die mit den Zahnungsabschnitten (36) der beiden Zahnstangen (35) kämmen,
oder
- eine stoffschlüssige Wirkverbindung aufweist, wobei ein erstes Zugmittel mit beiden Enden an dem ersten Wirkabschnitt (W1) befestigt ist und der erste Kraftübertrager (16a) von dem ersten Zugmittel umschlungen wird, und ein zweites Zugmittel mit beiden Enden an dem zweiten Wirkabschnitt (W2) befestigt ist und der zweite Kraftübertrager (16b) von dem zweiten Zugmittel umschlungen wird.

9. Chirurgisches Instrument (1), das einen Schaft (2), eine am proximalen Ende (3) des Schaftes (2) angeordnete Betätigungseinheit (4) und ein am distalen Ende (5) des Schaftes (2) angeordnetes Werkzeug (7) mit einer mittels einer distalen Abwinkelungsmechanik (9) abwinkelbaren Werkzeugspitze (6) aufweist, die durch eine mittels zweier Antriebe räumlich ausrichtbare Taumelscheibe (14) steuerbar ist, **dadurch gekennzeichnet, dass**
das chirurgische Instrument (1) ein Lenkgetriebe (13) nach zumindest einem der Ansprüche 1 bis 8 zur räumlichen Ausrichtung der Taumelscheibe (14) aufweist.

10. Chirurgisches Instrument (1) nach Anspruch 9,
**dadurch gekennzeichnet, dass**
ein Betätigungselement (8) axial verschiebbar in dem Schaft (2) gelagert ist und proximalseitig mit der Betätigungseinheit (4) in Wirkverbindung steht, und/oder die distalen Abwinkelungsmechanik (9) der abwinkelbaren Werkzeugspitze (6) aus an dem distalen Ende (5) des Schaftes (2) angeordneten Schwenkgliedern (11) besteht, die über in Längsrichtung des Schaftes (2) verlaufende Lenkdrähte (12) mit dem Lenkgetriebe (13) verbunden sind, die an der Taumelscheibe (14) befestigt sind.

## Claims

1. A steering gear (13) for a surgical instrument (1), wherein the steering gear (13) has a swash plate (14), wherein the steering gear (13) can be arranged at the proximal end (3) of a shaft (2) which defines a longitudinal axis (B) and has a bending mechanism (9) at the distal end (5), wherein the steering gear (13) has two motorised drives and is designed to spatially align the swash plate (14) via the adjustment angles of the two drives, which swash plate is designed to control the distal bending mechanism (9) of the surgical instrument (1),
wherein
the swash plate (14) is arranged in a steering ring (19), **characterised in that**
the first of the two motorised drives has a first drive shaft (17a) which is driven by a first motor (17) and is directly operatively connected to the steering ring (19) via a first force transmitter (16a), by the first force transmitter (16a) directly contacting the steering ring (19) at a first operative section (W1), wherein the first force transmitter (16) is arranged on the first drive shaft (17a), which defines a first drive axis (C), and
the second of the two motorised drives has a second drive shaft (17b) which is driven by a second motor (17') and is directly operatively connected to the steering ring (19) via a second force transmitter (16b), by the second force transmitter (16b) directly contacting the steering ring (19) at a second operative section (W2), wherein the second force transmitter (16b) is arranged on the second drive shaft (17b), which defines a second drive axis (C'),
wherein the steering ring (19) is cardanically mounted about a cardanic centre (Z) and is formed in the shape of a spherical segment at least on the first and the second operative section (W1, W2), wherein the spherical segmentshaped operative sections (W1, W2) have a common spherical centre point which corresponds to the cardanic centre (Z), in which a first tilt axis (E), which is defined by a contact point (K1) of the first force transmitter (16a) with the steering ring (19) and the cardanic centre (Z), and a second tilt axis (E'), which is defined by a contact point (K2) of the second force transmitter (16b) with the steering ring (19) and the cardanic centre (Z), intersect at right angles.

2. The steering gear (13) according to claim 1,
**characterised in that**
the cardanic mounting of the steering ring (19) is provided by
a) the steering ring (19) being cardanically suspended on a first fastening device, wherein the first fastening device is a bracket (15) which
- is arranged on a section of the steering ring (19) which faces away from the operative sections (W1, W2), and
- at both ends is mounted on a housing (20) so as to be rotatable about a pivot axis (A) which runs perpendicular to the longitudinal axis (B) and perpendicular to the drive axes (C, C'), and
- has a receiving opening (22) in the centre,
wherein the steering ring (19) is mounted in the receiving opening (22) so as to be rotatable about an axis of rotation (D) which runs perpendicular to the pivot axis (A);
or by
b) the steering ring (19) being cardanically mounted on a main shaft (38) running coaxially to the longitudinal axis (B) of the shaft (2) via the swash plate (14), which is mounted in the steering ring (30) so as to be rotatable about the longitudinal axis (B) or is fixedly connected to the steering ring (19), wherein the swash plate (14) is pivotably mounted on a universal joint disc (37) via two bearing pins (39) arranged offset by 180° relative to one another, and the universal joint disc (28) is pivotably mounted on the main shaft (38) via two bearing pins (40) arranged offset by 180° relative to one another, and wherein the bearing pins (39, 40) of the swash plate (14) and the universal joint disc (37) are arranged offset by 90° relative to one another.

3. The steering gear (13) according to claim 1 or 2,
**characterised in that**
the operative connection provides a form-fitting engagement by
- the first and second force transmitters (16a, 16b) being rotatably mounted drive cones with a cone head (23) which has circumferentially a plurality of drive knobs (27) forming a knob ring (28), and wherein the steering ring (19) has, on each operative section (W1, W2), recesses (29) which mesh with the drive knobs (27) of the two knob rings (28), or
- the first and second force transmitters (16a, 16b) are worm shafts (34) which run parallel to the longitudinal axis (B), and wherein the steering ring (19) has, on each operative section (W1, W2), concentrically arranged recesses (29) which mesh with the worm shaft (34).

4. The steering gear (13) according to claim 3,
**characterised in that**
the drive knobs (27) have a hemispherical or semi-ellipsoidal shape,
and/or
the recesses (29) are rings arranged concentrically one inside another or are arc-shaped grooves.

5. The steering gear (13) according to claim 1 or 2,
**characterised in that**
the operative connection is provided by friction by
- the first and second force transmitters (16a, 16b) being rotatably mounted drive cones with a cone head (23) which has circumferentially a friction element, preferably a material which is applied around the cone head (23) and which provides a higher friction than the material of which the cone head (23) consists, or a friction band (26) which is applied circumferentially around the cone head (23) and which is a rubber band or a band with a roughened surface or a band with a toothing or knobs.

6. The steering gear (13) according to claim 1 or 2,
**characterised in that**
the operative connection of the first force transmitter (16a) with the steering ring (19) provides a form-fitting engagement and the operative connection of the second force transmitter (16b) with the steering ring (19) is provided by frictional engagement,
wherein the form-fitting engagement of the first force transmitter (16a) with the steering ring (19) is provided by the first force transmitter (16a)
- being a rotatably mounted drive cone with a cone head (23) which has circumferentially a plurality of drive knobs (27) forming a knob ring (28), wherein the steering ring (19) has, on the first operative section (W1), recesses (29) which mesh with the drive knobs (27) of the two knob rings (28), or
- being a worm shaft (34) which runs parallel to the longitudinal axis (B), and wherein the steering ring (19) has, on the first operative section (W1), concentrically arranged recesses (29) which mesh with the worm shaft (34),
and wherein the frictional engagement of the second force transmitter (16b) with the steering ring (19) is provided by
the second force transmitter (16b) being a rotatably mounted drive cone with a cone head (23), which has circumferentially a friction element which preferably
- is a material which is applied around the cone head (23) and which provides a higher friction than the material of which the cone head (23) consists, or
- is a friction band (26) which is applied circumferentially around the cone head (23) and which is a rubber band or a band with a roughened surface or a band with a toothing or knobs.

7. The steering gear (13) according to at least one of claims 3 to 6, **characterised in that**
the rotatably mounted drive cones each have a cone shaft (42) around which a bearing ring (25) is arranged adjacent to the cone head (23), and **in that** the housing (20) has a base (20a) with a respective through-opening for each drive axle (17a, 17b), wherein each through-opening has a first opening section (30) with a diameter which corresponds to a diameter of the bearing ring (25), and a second opening section (31) which is coaxial with the first opening section (30) and has a diameter which is smaller than the diameter of the first opening section (30) such that the housing (20) forms, in each through-opening, a shoulder (32), on which the respective bearing ring (25) rests.

8. The steering gear (13) according to claim 1 or 2,
**characterised in that**
the operative connection
- forms a linear drive, by the first and second force transmitters (16a, 16b) being toothed racks (35) with a toothing section (36) and wherein the steering ring (19) has, on its operative section (W), concentrically arranged recesses (29) which mesh with the toothing sections (36) of the two toothed racks (35),
or
- has a materially-bonded operative connection, wherein a first traction means is fastened at both ends to the first operative section (W1) and the first force transmitter (16a) is wrapped around by the first traction means, and a second traction means is fastened at both ends to the second operative section (W2) and the second force transmitter (16b) is wrapped around by the second traction means.

9. A surgical instrument (1) having a shaft (2), an actuation unit (4) arranged at the proximal end (3) of the shaft (2) and a tool (7) arranged at the distal end (5) of the shaft (2) with a tool tip (6) which can be angled by means of a distal bending mechanism (9) and which can be controlled by a swash plate (14) which can be spatially aligned by means of two drives,
**characterised in that**
the surgical instrument (1) has a steering gear (13) according to at least one of claims 1 to 8 for the spatial orientation of the swash plate (14).

10. The surgical instrument (1) according to claim 9,
**characterised in that**
an actuating element (8) is mounted so as to be axially displaceable in the shaft (2) and is operatively connected to the actuation unit (4) on the proximal side, and/or the distal bending mechanism (9) of the bendable tool tip (6) consists of swivel members (11) which are arranged at the distal end (5) of the shaft (2) and which are connected to the steering gear (13) via steering wires (12) which run in the longitudinal direction of the shaft (2) and which are fastened to the swash plate (14).

## Revendications

1. Appareil de direction (13) pour un instrument chirurgical (1), dans lequel l'appareil de direction (13) a un plateau oscillant (14), l'appareil de direction (13) peut être disposé à l'extrémité proximale (3) d'un arbre (2) qui définit un axe longitudinal (B) et a un mécanisme de flexion (9) à l'extrémité distale (5), dans lequel le mécanisme de direction (13) a deux entraînements motorisés et étant conçu de manière à orienter dans l'espace le plateau oscillant (14) par l'intermédiaire des angles de réglage des deux entraînements, ce plateau étant conçu de manière à commander un mécanisme de flexion distal (9) de l'instrument chirurgical (1),
dans lequel
le plateau oscillant (14) est disposé dans un anneau de direction (19), **caractérisé en ce que**
le premier des deux entraînements motorisés a un premier arbre d'entraînement (17a) qui est entraîné par un premier moteur (17) et qui est directement relié de manière opérationnelle à l'anneau de direction (19) par l'intermédiaire d'un premier transmetteur de force (16a), par le premier transmetteur de force (16a) en contact direct avec l'anneau de direction (19) au niveau d'une première section opérationnelle (W1), dans lequel le premier transmetteur de force (16) est disposé sur le premier arbre d'entraînement (17a), qui définit un premier axe d'entraînement (C), et
le deuxième des deux entraînements motorisés a un deuxième arbre d'entraînement (17b) qui est entraîné par un deuxième moteur (17') et qui est directement relié de manière opérationnelle à l'anneau de direction (19) par l'intermédiaire d'un deuxième transmetteur de force (16b), par le deuxième transmetteur de force (16b) en contact direct avec l'anneau de direction (19) au niveau d'une deuxième section opérationnelle (W2), dans lequel le deuxième transmetteur de force (16b) est disposé sur le deuxième arbre d'entraînement (17b), qui définit un deuxième axe d'entraînement (C'),
dans lequel l'anneau de direction (19) est monté de manière cardanique autour d'un centre cardanique (Z) et est en forme d'un segment sphérique au moins sur la première et la deuxième section opérationnelle (W1, W2), dans lequel les sections opérationnelles en forme de segments sphériques (W1, W2) ont un point central sphérique commun qui correspond au centre cardanique (Z), dans lequel un premier axe d'inclinaison (E), qui est défini par un point de contact (K1) du premier transmetteur de force (16a) avec l'anneau de direction (19) et le centre cardanique (Z), et un deuxième axe d'inclinaison (E'), qui est défini par un point de contact (K2) du deuxième transmetteur de force (16b) avec l'anneau de direction (19) et le centre cardanique (Z), se croisent à angle droit.

2. Appareil de direction (13) selon la revendication 1,
**caractérisé en ce que**
le montage cardanique de l'anneau de direction (19) est fourni par
a) l'anneau de direction (19) étant suspendu de manière cardanique sur un premier dispositif de fixation, dans lequel le premier dispositif de fixation est un support (15) qui
- est disposée sur une section de l'anneau de direction (19) qui est opposé aux sections opérationnelles (W1, W2), et
- est monté aux niveau deux extrémités sur un boîtier (20) de manière à pouvoir tourner autour d'un axe de pivotement (A) s'étendant perpendiculaire à l'axe longitudinal (B) et perpendiculaire aux axes d'entraînement (C, C'), et
- a une ouverture de réception (22) dans le centre,
dans laquelle l'anneau de direction (19) est monté dans l'ouverture de réception (22) de manière à pouvoir tourner autour d'un axe de rotation (D) s'étendant perpendiculaire à l'axe de pivotement (A) ;
ou par
b) l'anneau de direction (19) étant monté de manière cardanique sur un arbre principal (38) s'étendant coaxial à l'axe longitudinal (B) de l'arbre (2) par l'intermédiaire du plateau oscillant (14), qui est monté dans l'anneau de direction (30) de manière à pouvoir tourner autour de l'axe longitudinal (B) ou qui est relié de manière fixe à l'anneau de direction (19), dans lequel le plateau oscillant (14) est monté pivotant sur un disque de joint universel (37) par l'intermédiaire de deux goupilles d'appui (39) décalés de 180° l'une par rapport à l'autre, et dans lequel le disque de joint universel (28) est monté pivotant sur l'arbre principal (38) par l'intermédiaire de deux axes de roulement (40) décalés de 180° l'un par rapport à l'autre, et dans lequel les goupilles d'appui (39, 40) du plateau oscillant (14) et du disque de joint universel (37) sont décalés de 90° l'une par rapport à l'autre.

3. Appareil de direction (13) selon la revendication 1 ou 2,
**caractérisé en ce que**
la connexion opérationnelle fournit une mise en prise ajusté à la forme par
- les premier et deuxième transmetteurs de force (16a, 16b) étant des cônes d'entraînement montés rotatifs avec une tête de cône (23) qui a, sur sa circonférence, une pluralité de boutons d'entraînement (27) formant un anneau de boutons (28), et dans lequel l'anneau de direction (19) a, sur chaque section opérationnelle (W1, W2), des évidements (29) qui s'engrènent avec les boutons d'entraînement (27) des deux anneaux de boutons (28), ou
- les premier et deuxième transmetteurs de force (16a, 16b) sont des arbres à vis sans fin (34) s'étendant parallèles à l'axe longitudinal (B), et dans lequel l'anneau de direction (19) a, sur chaque section opérationnelle (W1, W2), des évidements (29) disposés concentriquement qui s'engrènent avec l'arbre à vis sans fin (34).

4. Appareil de direction (13) selon la revendication 3,
**caractérisé en ce que**
les boutons d'entraînement (27) ont une forme hémisphérique ou semiellipsoïdale,
et/ou
les évidements (29) sont des anneaux disposés concentriquement les uns dans les autres ou des rainures en forme d'arc.

5. Appareil de direction (13) selon la revendication 1 ou 2,
**caractérisé en ce que**
la connexion opérationnelle est fournie par frottement par
- les premier et deuxième transmetteurs de force (16a, 16b) étant des cônes d'entraînement montés rotatifs avec une tête de cône (23) qui a, sur sa circonférence, un élément de friction, de préférence un matériau qui est appliqué autour de la tête du cône (23) et qui offre une friction plus élevée que le matériau dont est constituée la tête du cône (23), ou une bande de friction (26) qui est appliquée circonférentiellement autour de la tête du cône (23) et qui est une bande en caoutchouc ou une bande avec une surface rugueuse ou une bande avec une denture ou des boutons.

6. Appareil de direction (13) selon la revendication 1 ou 2,
**caractérisé en ce que**
la connexion opérationnelle du premier transmetteur de force (16a) avec l'anneau de direction (19) fournit une mise en prise ajusté à la forme et la connexion opérationnelle du deuxième transmetteur de force (16b) avec l'anneau de direction (19) est fourni par une mise en prise par frottement,
dans lequel la mise en prise ajusté à la forme du premier transmetteur de force (16a) avec l'anneau de direction (19) est fournie par le premier transmetteur de force (16a)
- étant monté rotatif avec une tête de cône (23) qui a, sur sa circonférence, une pluralité de boutons d'entraînement (27) formant un anneau de boutons (28), dans lequel l'anneau de direction (19) a, sur la première section opérationnelle (W1), des évidements (29) qui s'engrènent avec les boutons d'entraînement (27) des deux anneaux de boutons (28), ou
- étant un arbre à vis sans fin (34) s'étendant parallèles à l'axe longitudinal (B), et dans lequel l'anneau de direction (19) a, sur la première section opérationnelle (W1), des évidements (29) disposés concentriquement qui s'engrènent avec l'arbre à vis sans fin (34),
et dans lequel mise en prise par frottement du deuxième transmetteur de force (16b) avec l'anneau de direction (19) est fournie par
le deuxième transmetteur de force (16b) étant un cône d'entraînement monté rotatif avec une tête de cône (23), qui a sur sa circonférence un élément de frottement qui de préférence
- est un matériau qui est appliqué autour de la tête de cône (23) et qui fournit une friction plus élevée que le matériau dont est constituée la tête de cône (23), ou
- est une bande de frottement (26) appliquée circonférentiellement autour de la tête du cône (23) et qui est une bande en caoutchouc ou une bande avec une surface rugueuse ou une bande avec une denture ou des boutons.

7. Appareil de direction (13) selon au moins l'une des revendications 3 à 6, **caractérisé en ce que**
les cônes d'entraînement montés rotatifs ont chacun un arbre de cône (42) autour duquel une bague de roulement (25) est disposée adjacente à la tête de cône (23), et **en ce que** le boîtier (20) a une base (20a) avec un orifice de passage respective pour chaque axe d'entraînement (17a, 17b), dans lequel chaque orifice de passage a une première section d'orifice (30) dont le diamètre correspond au diamètre de la bague de roulement (25), et une deuxième section d'orifice (31) qui est coaxiale avec la première section d'orifice (30) et dont le diamètre est inférieur au diamètre de la première section d'orifice (30), de sorte que le boîtier (20) se forme, dans chaque orifice de passage, une épaule (32), sur lequel repose la bague de roulement (25) respective.

8. Appareil de direction (13) selon la revendication 1 ou 2,
**caractérisé en ce que**
la connexion opérationnelle
- forme un entraînement linéaire, par les premier et deuxième transmetteurs de force (16a, 16b) étant des crémaillères (35) avec une section dentée (36) et dans lequel l'anneau de direction (19) a, sur sa section opérationnelle (W), des évidements (29) disposés concentriquement qui s'engrènent avec les sections dentées (36) des deux crémaillères (35),
ou
- a un entraînement linéaire lié de manière matériel, dans lequel un premier moyen de traction est fixé aux deux extrémités de la première section opérationnelle (W1) et le premier transmetteur de force (16a) est enveloppé par le premier moyen de traction, et un deuxième moyen de traction est fixé aux deux extrémités de la deuxième section opérationnelle (W2) et le deuxième transmetteur de force (16b) est enveloppé par le deuxième moyen de traction.

9. Instrument chirurgical (1) ayant un arbre (2), une unité d'actionnement (4) disposée à l'extrémité proximale (3) de l'arbre (2) et un outil (7) disposé à l'extrémité distale (5) de l'arbre (2) avec une pointe d'outil (6) qui peut être inclinée au moyen d'un mécanisme de flexion distale (9) et qui peut être contrôlée par un plateau oscillant (14) qui peut être aligné dans l'espace au moyen de deux entraînements,
**caractérisé en ce que**
l'instrument chirurgical (1) a un appareil de direction (13) selon au moins l'une des revendications 1 à 8 pour l'orientation spatiale du plateau oscillant (14).

10. Instrument chirurgical (1) selon la revendication 9,
**caractérisé en ce que**
un élément d'actionnement (8) est monté de manière à pouvoir être déplacé axialement dans l'arbre (2) et est relié de manière opérationnelle à l'unité d'actionnement (4) sur le côté proximal, et/ou le mécanisme de flexion distale (9) de la pointe de l'outil pliable (6) constitue des éléments pivotants (11) qui sont disposés à l'extrémité distale (5) de l'arbre (2) et qui sont reliés à l'appareil de direction (13) par des fils de direction (12) s'étendant dans la direction longitudinale de l'arbre (2) et qui sont fixés au plateau oscillant (14).
